# EUROPÄISCHE PATENTSCHRIFT

(11) **EP 2 320 864 B1**
(45) Veröffentlichungstag und Bekanntmachung des Hinweises auf die Patenterteilung: **16.08.2017**
(21) Anmeldenummer: 09782551.7
(22) Anmeldetag: 03.09.2009
(51) Int. Cl.: A61K 8/44, A61K 8/64, A61K 8/73, A61Q 5/06, A61Q 5/10, A61Q 5/00

(54) **HAARBEHANDLUNGSMITTEL MIT CHITOSAN SUCCINAMID**
HAIR PREPARATION COMPRISING CHITOSAN SUCCINAMIDE
PRODUIT DE TRAITEMENT CAPILLAIRE CONTENANT DU SUCCINAMIDE DE CHITOSANE

(30) Priorität: 11.09.2008 DE 102008046882
(43) Veröffentlichungstag der Anmeldung: 18.05.2011
(73) Patentinhaber: Henkel AG & Co. KGaA, 40589 Düsseldorf (DE)
(72) Erfinder: ZIRWEN, Sabrina, 22089 Hamburg (DE); KLEEN, Astrid, 20457 Hamburg (DE)
(86) Internationale Anmeldenummer: PCT/EP2009/061390
(87) Internationale Veröffentlichungsnummer: WO 2010/029007

(56) Entgegenhaltungen:
- EP-A- 1 813 258
- WO-A-2004/087086
- DE-A1-102007 039 012
- JP-A- 2007 284 380

## Beschreibung

Die Erfindung betrifft ein Mittel zur Farb- und/oder dauerhaften Formveränderung von keratinhaltigen Fasern, insbesondere menschlichen Haaren, welches in einem kosmetischen Träger mindestens eine farb- und/oder formverändernde Verbindung sowie ein N-Acyl-Derivat des Chitosans und mindestens einen Wirkstoff, der ausgewählt wird aus L-Serin, D-Serin und D/L-Serin, enthält. Weiterhin betrifft die Erfindung ein Verfahren von solchen Mitteln auf keratinhaltigen Fasern. Darüber hinaus betrifft die Erfindung die Verwendung dieses Mittels zur Färbung und/oder dauerhaften Formveränderung.

Die Veränderung von Form und Farbe der Haare stellt einen wichtigen Bereich der modernen Kosmetik dar. Zur Bereitstellung farbverändernder kosmetischer Mittel, insbesondere für die Haut oder keratinhaltige Fasern wie beispielsweise menschliche Haare, kennt der Fachmann je nach Anforderungen an die Färbung diverse Färbesysteme.

In jüngster Zeit wurden so genannte Kombinationspräparate entwickelt, um den Aufwand der üblichen mehrstufigen Verfahren, insbesondere bei der direkten Anwendung durch Verbraucher, zu verringern. Diese Präparate enthalten neben den üblichen Komponenten, beispielsweise zur Färbung der Haare, zusätzlich Wirkstoffe, die früher den Haarnachbehandlungsmitteln vorbehalten waren. Der Konsument spart somit einen Anwendungsschritt; gleichzeitig wird der Verpackungsaufwand verringert, da ein Produkt weniger gebraucht wird. Dennoch lassen auch diese Mittel, insbesondere auf schwer zu pflegenden Fasern, wie beispielsweise japanischem Haar, noch einige Wünsche hinsichtlich der pflegenden Eigenschaften offen.

Die im Rahmen derartiger Kombinationspräparate einsetzbaren Wirkstoffe müssen insbesondere hinsichtlich ihrer Stabilität hohen Ansprüchen genügen, da die Färbecremes beziehungsweise Wellmittel üblicherweise einen hohen pH-Wert und die Oxidationsmittelzubereitungen einen niedrigen pH-Wert aufweisen. Ferner sind Unverträglichkeiten der verschiedenen Wirkstoffe untereinander und somit eine geringe Lagerstabilität zu vermeiden.

Insbesondere oxidative Haarfärbemittel sind trotz ihrer vorteilhaften Färbeeigenschaften für den Anwender mit Nachteilen behaftet. Einerseits führt der Einsatz der Oxidationsmittel zur Ausfärbung respektive Entwicklung der eigentlichen Färbung zu Schädigungen in der Haarstruktur und auf der Haaroberfläche. Das Haar wird brüchig, seine Elastizität lässt nach und die Kämmbarkeit nimmt ab. Andererseits benötigen oxidative Färbemittel in der Regel einen basischen pH-Wert zur Ausfärbung, insbesondere zwischen pH 9,0 und pH 10,5. Das basische Milieu stellt jedoch einen weiteren Grund der Schädigung für das Haar und dessen Struktur dar, der ebenfalls mit gesteigerter Anwendungszeit an Bedeutung gewinnt. Die Spreizung der äußeren Schuppenschicht führt außerdem zu einem unangenehmen Oberflächenempfinden der Haare und damit zu einer verschlechterten Kämmbarkeit im Nass- und Trockenzustand. Viele gebräuchliche Färbemittel enthalten zudem einen hohen Anteil an anionischen, grenzflächenaktiven Verbindungen. Solche anionischen Tenside neigen aber dazu, die keratinische Faser in ihrer natürlichen Feuchtigkeit zu beeinträchtigen und die Faser so auszutrocknen.

Weiterhin wird der basische pH-Wert häufig mit Ammoniak als Alkalisierungsmittel eingestellt. Durch Ammoniak kann es neben zusätzlichen Schädigungen des Haares zu Reizungen von Augen oder Kopfhaut kommen, wodurch Sensibilisierungen oder gar allergische Reaktionen hervorgerufen werden können. Darüber hinaus besitzen solche Färbemittel einen intensiven, unangenehmen Geruch, der schlimmstenfalls auch zu Reizungen der Nasenschleimhaut führen kann. Außerdem kann auch die Produktion und Lagerung von Ammoniak-haltigen Färbemitteln mit Problemen hinsichtlich Handhabbarkeit und Stabilität verbunden sein.

Häufige Haarwäsche, insbesondere durch den Einsatz anionischer Tenside in Shampoos, führt ebenfalls zu Beeinträchtigungen der Oberfläche und der Struktur von Haaren. Nicht zuletzt wird die Haarstruktur jedoch auch durch äußere Umwelteinflüsse in Mitleidenschaft gezogen. Hierzu sind mechanische und thermische Einwirkungen, wie Kämmen und Fönen, zu zählen. Ebenso tragen Wettereinflüsse, wie Wind, Regen und UV-Strahlung im Sonnenlicht, und zusätzliche äußere Belastungen, wie beispielsweise chloriertes Schwimmbadwasser oder Schweiß, zur Schädigung der Haarstruktur und der Haaroberfläche bei.

Ein weiterer Nachteil der bisher eingesetzten Mittel liegt in dem deutlichen Feuchtigkeitsverlust der Fasern und der Kopfhaut, der mit der Behandlung einhergeht, begründet, der die Fasern und gegebenenfalls die Kopfhaut langfristig schädigen kann.

Es besteht daher weiterhin ein Bedarf an pflegenden Wirkstoffen für die farb- und/oder formverändernde Behandlung von Fasern, die darüber hinaus das Austrocknen der Fasern und der Kopfhaut vermeiden. Aufgabe der vorliegenden Erfindung ist es daher, ein farb- und/oder formveränderndes Mittel zur Verfügung zu stellen, durch welches die oben genannten Nachteile gebräuchlicher farb- und/oder formverändernden Mittel herabgesenkt werden. Die farb- und/oder formverändernden Mittel sollen das Haar schützen und damit eine verringerte Schädigung des Haares bewirken. Gerade angegriffene und durch äußere Einflüsse vorgeschädigte Haare sollen durch Färbung möglichst wenig zusätzliche Schädigung erfahren. Insbesondere Schutz vor oxidativen Schädigungen der Haarstruktur und der Haaroberfläche soll durch die farb- und/oder formverändernden Mittel erzielt werden. Dazu ist es wünschenswert, wenn das farb- und/oder formverändernden Mittel feuchtigkeitsregulierend und/oder strukturierend für Haar und Haut wirkt und gleichzeitig das Resultat der farb- und/oder formverändernden Behandlung in seinen Eigenschaften, wie beispielsweise den Echtheitseigenschaften, verbessert. Besonders wünschenswert sind pflegende Eigenschaften der Mittel, so dass der Anwender auf den Einsatz zusätzlicher Konditionier- und Nachbehandlungsmittel verzichten kann. Weiterhin sollen die Mittel möglichst einen in einem physiologisch wenig beeinträchtigenden pH-Bereich eingesetzt werden, insbesondere in einem neutralen pH-Bereich. Schließlich ist es wünschenswert, farb- und/oder formverändernde Mittel bereitzustellen, bei denen das Reizpotential, insbesondere hervorgerufen durch den Zusatz von Ammoniak, möglichst niedrig ist.

EP 1 813 258 A2 betrifft Aufhell- und/oder Färbemittel mit reduziertem Irritationspotential, welche Valin und Chitosan bzw. Chitosanderivate enthalten.

In DE 10 2007 039 012 A1 werden Haarbehandlungsmittel mit Orangenöl offenbart, die als optionale Bestandteile auch Aminosäuren und Chitosanderivate enthalten können.

In nicht vorhersehbarer Weise konnte nun gefunden werden, dass farb- und/oder formverändernde Mittel, die neben einer farb- und/oder formverändernden Komponente mindestens ein N-Acyl-Derivat des Chitosans sowie einen Wirkstoff auf Aminosäure-Basis enthalten, die oben genannten Nachteile vermeiden. Bedingt durch die Schutzwirkung bei Anwendung des erfindungsgemäßen Mittels kann die Haarschädigung hierdurch minimiert werden oder sogar eine Haarpflege erzielt werden.

Ein erster Gegenstand der Erfindung ist daher ein Mittel zur Farb- und/oder dauerhaften Formveränderung keratinischer Fasern, insbesondere menschlicher Haare, enthaltend in einem kosmetischen Träger mindestens eine farb- und/oder formverändernde Verbindung und zusätzlich mindestens ein N-Acyl-Derivat des Chitosans und mindestens einen Wirkstoff, der ausgewählt wird aus der Gruppe, die gebildet wird aus L-Serin, D-Serin und D/L-Serin, wobei das N-Acyl-Derivat des Chitosans aus Glucosamin, N-Acetylglucosamin und N-Succinylglucosamin aufgebaut ist.

Unter keratinischen Fasern oder auch Keratinfasern sind dabei Pelze, Wolle, Federn und insbesondere menschliche Haare zu verstehen. Obwohl die erfindungsgemäßen Mittel in erster Linie zum Färben von Keratinfasern geeignet sind, steht prinzipiell einer Verwendung auch auf anderen Gebieten nichts entgegen.

Die erfindungsgemäßen Mittel enthalten die Wirkstoffe in einem kosmetischen Träger. Dieser kosmetische Träger ist im Sinne der Erfindung ist wässrig, alkoholisch oder wässrig-alkoholisch. Zum Zwecke der Haarfärbung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind. Unter wässrig-alkoholischen Trägern sind im Sinne der vorliegenden Erfindung wasserhaltige Zusammensetzungen, enthaltend 3 bis 70 Gew.-% eines C₁-C₄-Alkohols, bezogen auf das Gesamtgewicht der Anwendungsmischung, insbesondere Ethanol bzw. Isopropanol, zu verstehen. Die erfindungsgemäßen Mittel können zusätzlich weitere organische Lösungsmittel, wie beispielsweise 4-Methoxybutanol, Ethyldiglykol, 1,2-Propylenglykol, n-Propanol, n-Butanol, n-Butylenglykol, Glycerin, Diethylenglykolmonoethylether, und Diethylenglykolmono-n-butylether, enthalten. Bevorzugt sind dabei alle wasserlöslichen organischen Lösungsmittel.

Ein wässriger Träger enthält im Sinne der Erfindung mindestens 30 Gew.-%, insbesondere mindestens 50 Gew.-% Wasser, bezogen auf das Gesamtgewicht der Anwendungsmischung.

Als ersten wesentlichen Inhaltsstoff enthält das erfindungsgemäße Mittel mindestens ein N-Acyl-Derivat des Chitosans, das aus Glucosamin, N-Acetylglucosamin und N-Succinylglucosamin aufgebaut ist.

Chitosan wird durch alkalische Deacetylierung von Chitin erhalten, welches überwiegend in wirbellosen Tieren vorkommt, wie zum Beispiel im Außenskelett von Gliederfüßern oder in Strukturporteinen von Mollusken. Chitin besteht aus Ketten von β-1,4-glycosidisch verknüpften N-Acetyl-D-glucosamin-(NAG-)Resten und kann deshalb auch als Cellulose-Derivat angesehen werden. Chitin ist selbst besitzt ein Molekulargewicht von einigen 100.000 bis > 1.000.000 g/Mol und unlöslich in Wasser, Alkohol, Ether, Aceton und Chloroform sowie in verdünnten Säuren und konzentrierten Laugen.

Zur Chitosangewinnung wird üblicherweise von natürlichem Chitin ausgegangen. Überwiegend wird Chitosan über ein chemisches Verfahren aus natürlichen Quellen, vor allem aus Krabbenschalen, gewonnen. Diese werden vermahlen, deproteiniert (Extraktion mit verdünnter Natronlauge), demineralisiert und entfärbt (z.B. mit Oxidationsmitteln). Nach einer alkalischen oder enzymatischen Deacetylierung und einem Waschschritt wird Chitosan erhalten.

Der Übergang von Chitin zu Chitosan ist dabei nicht scharf definiert. Üblicherweise wird von Chitosan gesprochen, wenn ein Deacetylisierungsgrad von >40-50 % erreicht ist, und die Verbindung in organischen Säuren löslich ist.

Üblicherweise enthalten Chitosan und Chitosanderivate daher neben D-Glucosamin-Einheiten auch noch einen signifikanten Anteil an N-Acetyl-D-glucosamin-Resten in der Polymerkette. Unter N-Acyl-Derivat von Chitosan soll daher im Rahmen dieser Erfindung ein vom Acetyl-Rest verschiedener Acyl gelten. Chitin selbst ist daher kein erfindungsgemäßes N-Acyl-Derivat des Chitosan im Sinne der vorliegenden Erfindung.

Die erfindungsgemäßen N-Acyl-Derivate des Chitosans sind zugänglich durch die Umsetzung von Chitosan mit Carbonsäurehalogeniden, Carbonsäureanhydriden oder durch geeignete Methoden aus Carbonsäureestern oder Carbonsäuren selbst, wobei üblicherweise eine Aktivierung der Carbonsäure selbst die Umsetzung mit derAmin-Funktion der Glucosamin-Einheit erleichtert. Geeignete Methoden sind dem Fachmann wohl bekannt und umfassen enzymatische wie klassische Amidinierungsreaktionen, wie beispielsweise beschrieben in J. March, Advanced Organic Chemistry: Reactions, Mechanisms and Structure, 4th Edition, J. Wiley & Sons, New York, 1992.

Eine Ausführungsführung der Erfindung liegt vor, wenn das N-Acyl-Derivat ein Umsetzungsprodukt von Chitosan mit Bernsteinsäureanhydrid darstellt. Ein erfindungsgemäßes N-Acyl-Derivat des Chitosans ist daher aus Glucosamin, N-Acetylglucosamin und N-Succinylglucosamin aufgebaut.

Besonders bevorzugte N-Acyl-Derivate des Chitosans enthalten Glucosamin zu ca. 35 bis 50 Gew.-%, bevorzugt zu 40 bis 45 Gew.-%, N-Acetylglucosamin zu ca. 5 bis 25 Gew.-%, bevorzugt zu 10 bis 20 Gew.-% und N-Succinylglucosamin zu ca. 35 bis 50 Gew.-%, bevorzugt zu 40 bis 45 Gew.-%, jeweils bezogen auf das Gesamtgewicht des Chitosanderivats.

Das Molekulargewicht des N-Acyl-Derivat beträgt bevorzugt kleiner 1.000.000 g/Mol, besonders bevorzugte N-Acyl-Chitosan-Derivate besitzen ein Molekulargewicht von 100.00 bis 500.000 g/Mol.

Ein Bespiel eines erfindungsgemäß bevorzugt eingesetzten N-Acyl-Chitosan-Derivats wird unter dem Handelsnamen Hydrasens® von der Firma Soliance vertrieben.

Weiterhin enthält das erfindungsgemäße Mittel zusätzlich mindestens einen weiteren Wirkstoff, der ausgewählt wird aus der Gruppe, die gebildet wird L-Serin, D-Serin und D/L-Serin.

L-Serin, D-Serin und D/L-Serin können den erfindungsgemäßen Mitteln bevorzugt in freier Form zugegeben werden. In einer Reihe von Fällen ist es jedoch auch vorteilhaft, insbesondere die Aminosäuren in Salzform einzusetzen. Bevorzugte Salze sind dann die Verbindungen mit Halogenwasserstoffsäuren oder Schwefelsäure, insbesondere die Hydrochloride, die Hydrobromide und die Sulfate.

Der zusätzliche Wirkstoff, ausgewählt aus L-Serin, D-Serin und D/L-Serin , ist in den erfindungsgemäßen Mitteln bevorzugt in Mengen von 0,01 bis 10 Gew.-%, insbesondere von 0,05 bis 5 Gew.-%, bezogen auf das Gesamtgewicht der Anwendungsmischung, enthalten.

In einer bevorzugten Ausführungsform der vorliegenden Erfindung enthält das Mittel zur Farb- und/oder dauerhaften Formveränderung keratinischer Fasern zur weiteren Verbesserung des Pflegezustands der keratinischen Faser zusätzlich mindestens einen Extrakt aus einer Pflanze der Gattung Aloe.

Prinzipiell können erfindungsgemäß alle in der Natur vorkommenden Pflanzen, die zu der Gattung Aloe zählen, eingesetzt werden. In der Literatur werden derzeit über 300 verschiedene Pflanzenarten beschrieben. Als bevorzugte Arten können *Aloe mutabilis, Aloe melanocantha, Aloe manchii, Aloe pearsonii*, *Aloe comptonii, Aloe mitriformis, Aloe distans, Aloe arenicola, Aloe volkensii, Aloe petrophylla, Aloe ferox, Aloe africana, Aloe globiligemma , Aloe perry, Aloe visckensii, Aloe vera, Aloe lettyae, Aloe capensis, Aloe barbadensis, Aloe socetriis, Aloe curacao, Aloe candelabrum, Aloe excelsa, Aloe cameronii, Aloe sessiliflora, Aloe reitzii, Aloe aculeate, Aloe marlothii, Aloe utyhei sensis, Aloe dolomitica, Aloe castanea, Aloe vanlabemii, Aloe alooides, Aloe gerstueri* und *Aloe petricola* genannt werden.

Erfindungsgemäß besonders bevorzugt sind Zubereitungen, die aus *Aloe capensis* oder *Aloe barbadensis* gewonnen werden. Erfindungsgemäß besonders bevorzugt sind Zubereitungen, die aus *Aloe barbadensis,* der so genannten Echten Aloe oder auch Aloe vera, gewonnen werden.

Hinsichtlich der Art des Extrakts aus Aloe bestehen prinzipiell keinerlei Beschränkungen. So können sowohl die Blätter als auch die Blüten und Samen der Aloe-Pflanzen als Basis für diese Zubereitung dienen. Als erfindungsgemäß besonders bevorzugt haben sich Zubereitungen erwiesen, die aus den Blättern der Aloe-Pflanzen gewonnen werden.

Auch hinsichtlich der Art und Weise, wie die erfindungsgemäße Zubereitung aus den Pflanzenbestandteilen gewonnen wird, bestehen prinzipiell keine Einschränkungen. So kann beispielsweise das in den Pflanzen enthaltene Gel direkt in der Form eingesetzt werden, in der es bei Verletzung der Aloe-Blätter auftritt.

Es können auch Zubereitungen eingesetzt werden, die mittels mechanischer und/oder chemischer Techniken aus dem Pflanzen gewonnen werden. Dazu können die Pflanzenbestandteile in einem fakultativen ersten Schritt mechanisch zerkleinert werden. Als Beispiele für mechanische Zerkleinerungsmethoden seien Zerschneiden, Pürieren und Zerpressen genannt.

Im zweiten wesentlichen Schritt können die Zubereitungen dann in einer ersten Ausführungsform mittels mechanischer Trennmethoden aus dem Pflanzenbestandteilen gewonnen werden, die auf der Ausnutzung von mechanischen Kräften, wie beispielsweise Schwerkraft, Zentrifugalkraft, Druck oder Vakuum, beruhen. Zu diesen gehören beispielsweise Dekantieren, Filtration, Sedimentation, Ultrafiltration und Zentrifugieren.

In einer weiteren Ausführungsform der vorliegenden Erfindung werden Zubereitungen eingesetzt, die mittels chemischer Trennmethoden, beispielsweise einer Extraktion oder einem chromatographischen Verfahren, aus den Pflanzenbestandteilen gewonnen werden. Im Rahmen dieser Ausführungsform sind Extrakte besonders bevorzugt. Als Extraktionsmittel zur Herstellung der genannten Pflanzenextrakte können Wasser, Alkohole, Ester sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/Propylenglykol haben sich als besonders geeignet erwiesen. Es hat sich dabei als besonders geeignet erwiesen, wenn diese Extraktionsmittel in einem Verhältnis von 1:10 bis 10:1 eingesetzt werden. Unter den Estern kann es erfindungsgemäß bevorzugt sein, Ester von kurzkettigen Carbonsäuren und kurzkettigen Alkoholen einzusetzen. Hierzu zählen beispielsweise Ethylacetat, Isopropylacetat, Ethylpropionat. Weiterhin vorteilhaft können auch Ester langkettiger, gegebenenfalls verzweigter Fettsäureneingesetzt werden. Hier hat sich Isopropylmyristat als besonders geeignet herausgestellt. Weiterhin kann es erfindungsgemäß bevorzugt sein, Extrakte einzusetzen, die vor der Anwendung zumindest teilweise entfärbt wurden. Dies kann beispielsweise durch Nutzung von Aktivkohle erfolgen.

Ein erfindungsgemäß besonders bevorzugter Extrakt von Aloe-Pflanzen ist aus den Blättern von Aloe Barbadensis gewonnener Extrakt in Isopropylmyristat. Ein solcher Extrakt wird beispielsweise unter dem Handelsnamen Aloe Vera Extrakt von der Firma Worlée, Hamburg, vertrieben.

Besonders vorteilhaft wird der Extrakt der Aloe zu 0,1 bis 8 Gew.-%, insbesondere 0,2 bis 5,0 Gew.-%, jeweils bezogen auf das anwendungsbereite Mittel, eingesetzt.

In einer bevorzugten Ausführungsform enthält das erfindungsgemäße Mittel zur Farbveränderung keratinischer Fasern eine farbgebende Komponente. Die farbgebende Komponente wird bevorzugt ausgewählt (1) aus mindestens einem Oxidationsfarbstoffvorprodukt und/oder (2) aus Oxofarbstoffvorprodukten und/oder (3) aus mindestens einer Vorstufe naturanaloger Farbstoffe und/oder (4) aus mindestens einem direktziehenden Farbstoff und/oder (5) mindestens einem Naturfarbstoff.

In einer ersten Ausführungsform werden die farbverändernden Komponenten ausgewählt aus Oxidationsfarbstoffvorprodukten. Die Oxidationsfarbstoffvorprodukte werden bevorzugt in einer Menge von 0,005 bis 20 Gew.-%, bevorzugt von 0,05 bis 5 Gew.-% und besonders bevorzugt von 0,1 bis 5 Gew.-%, jeweils bezogen auf das anwendungsbereite Oxidationsfärbemittel, verwendet.

In einer bevorzugten Ausführungsform enthalten die erfindungsgemäßen Mittel mindestens ein Oxidationsfarbstoffvorprodukt vom Entwicklertyp und/oder Kupplertyp enthalten. Vorzugsweise enthalten die erfindungsgemäßen Färbemittel mindestens ein Oxidationsfarbstoffvorprodukt vom Entwicklertyp und mindestens ein Oxidationsfarbstoffvorprodukt vom Kupplertyp. Die Entwickler- und Kupplerkomponenten werden üblicherweise in freier Form eingesetzt. Bei Substanzen mit Aminogruppen kann es aber bevorzugt sein, sie in Salzform, insbesondere in Form der Hydrochloride und Hydrobromide oder der Sulfate einzusetzen.

Nachfolgend werden erfindungsgemäße Entwicklerkomponenten eingehender vorgestellt. Besonders bevorzugt sind p-Phenylendiaminderivate. Besonders bevorzugte p-Phenylendiamine werden ausgewählt aus einer oder mehrerer Verbindungen der Gruppe, die gebildet wird, aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-Bis-(2-hydroxyethyl)-p-phenylendiamin, 4-N,N-Bis-(2-hydroxyethyl)-amino-2-methylanilin, 4-N,N-Bis-(2-hydroxyethyl)-amino-2-chloranilin, 2-(2-Hydroxyethyl)-p-phenylendiamin, 2-(1,2-Dihydroxyethyl)-p-phenylendiamin, 2-Fluor-p-phenylendiamin, 2-Isopropyl-p-phenylendiamin, N-(2-Hydroxypropyl)-p-phenylendiamin, 2-Hydroxymethyl-p-phenylendiamin, N,N-Dimethyl-3-methyl-p-phenylendiamin, N-Ethyl-N-2-hydroxyethyl-p-phenylendiamin, N-(2,3-Dihydroxypropyl)-p-phenylendiamin, N-(4'-Aminophenyl)-p-phenylendiamin, N-Phenyl-p-phenylendiamin, 2-(2-Hydroxyethyloxy)-p-phenylendiamin, 2-Methoxymethyl-p-phenylendiamin, 2-(2-Acetylaminoethyloxy)-p-phenylendiamin, N-(2-Methoxyethyl)-p-phenylendiamin, N-(4-Amino-3-methylphenyl)-N-[3-(1H-imidazol-1-yl)propyl]amin, 5,8-Diaminobenzo-1,4-dioxan sowie ihren physiologisch verträglichen Salzen. Erfindungsgemäß ganz besonders bevorzugte p-Phenylendiaminderivate sind ausgewählt aus mindestens einer Verbindung der Gruppe p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, N-(4-Amino-3-methylphenyl)-N-[3-(1 H-imidazol-1-yl)propyl]amin, 2-Methoxymethyl-p-phenylendiamin sowie den physiologisch verträglichen Salzen dieser Verbindungen.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind. Bevorzugte zweikernige Entwicklerkomponenten werden insbesondere aus mindestens einer der folgenden Verbindungen ausgewählt: N,N'-Bis-(2-hydroxyethyl)-N,N'-bis-(4'-aminophenyl)-1,3-diamino-propan-2-ol, N,N'-Bis-(2-hydroxyethyl)-N,N'-bis-(4'-aminophenyl)-ethylendiamin, N,N'-Bis-(4'-aminophenyl)tetramethylendiamin, N,N'-Bis-(2-hydroxyethyl)-N,N'-bis-(4'-aminophenyl)-tetramethylendiamin, N,N'-Bis-(4-(methylamino)phenyl)tetramethylendiamin, N,N'-Diethyl-N,N'-bis-(4'-amino-3'-methylphenyl)ethylendiamin, Bis-(2-hydroxy-5-aminophenyl)methan, N,N'-Bis-(4'-aminophenyl)-1,4-diazacycloheptan, N,N'-Bis-(2-hydroxy-5-aminobenzyl)piperazin, N-(4'-Aminophenyl)-p-phenylendiamin und 1,10-Bis-(2',5'-diaminophenyl)-1,4,7,10-tetraoxadecan sowie ihre physiologisch verträglichen Salze. Ganz besonders bevorzugte zweikernige Entwicklerkomponenten werden ausgewählt unter N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan oder eines der physiologisch verträglichen Salze dieser Verbindungen.

Weiterhin kann es erfindungsgemäß bevorzugt sein, als Entwicklerkomponente ein p-Aminophenolderivat oder eines seiner physiologisch verträglichen Salze einzusetzen. Bevorzugte p-Aminophenole sind insbesondere p-Aminophenol, N-Methyl-p-aminophenol, 4-Amino-3-methylphenol, 4-Amino-3-fluorphenol, 2-Hydroxymethylamino-4-aminophenol, 4-Amino-3-hydroxymethylphenol, 4-Amino-2-(2-hydroxyethoxy)phenol, 4-Amino-2-methylphenol, 4-Amino-2-hydroxymethylphenol, 4-Amino-2-methoxymethyl-phenol, 4-Amino-2-aminomethylphenol, 4-Amino-2-(2-hydroxyethyl-aminomethyl)-phenol, 4-Amino-2-(1,2-dihydroxyethyl)-phenol, 4-Amino-2-fluorphenol, 4-Amino-2-chlorphenol, 4-Amino-2,6-dichlorphenol, 4-Amino-2-(diethylaminomethyl)phenol sowie ihre physiologisch verträglichen Salze. Ganz besonders bevorzugte Verbindungen sind p-Aminophenol, 4-Amino-3-methylphenol, 4-Amino-2-aminomethylphenol, 4-Amino-2-(1,2-dihydroxyethyl)phenol und 4-Amino-2-(diethylaminomethyl)phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die Entwicklerkomponente ausgewählt sein aus heterocyclischen Entwicklerkomponenten, wie beispielsweise aus Pyrimidinderivaten, Pyrazolderivaten, Pyrazolopyrimidin-Derivaten bzw. ihren physiologisch verträglichen Salzen. Bevorzugte Pyrimidin-Derivate sind insbesondere die Verbindungen 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin. Bevorzugte Pyrazol-Derivate sind insbesondere die Verbindungen, die ausgewählt werden unter 4,5-Diamino-1-methylpyrazol, 4,5-Diamino-1-(2-hydroxyethyl)pyrazol, 3,4-Diaminopyrazol, 4,5-Diamino-1-(4'-chlorbenzyl)pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert-butyl-1-methylpyrazol, 4,5-Diamino-1-tert.-butyl-3-methylpyrazol, 4,5-Diamino-1-(2-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(2-aminoethyl)amino-1,3-dimethylpyrazol, sowie deren physiologisch verträglichen Salze. Unter den Pyrazolo[1,5-a]pyrimidinen kann man insbesondere nennen: Pyrazolo[1,5-a]pyrimidin-3,7-diamin; 2,5-Dimethyl-pyrazolo[1,5-a]pyrimidin-3,7-diamin; Pyrazolo[1,5-a]pyrimidin-3,5-diamin; 2,7-Dimethyl-pyrazolo[1,5-a]pyrimidin-3,5-diamin; 3-Aminopyrazolo[1,5-a]pyrimidin-7-ol; 3-Aminopyrazolo[1,5-a]pyrimidin-5-ol; 2-(3-Aminopyrazolo[1,5-a]-pyrimidin-7-ylamino)-ethanol; 2-(7-Aminopyrazolo[1,5-a]pyrimidin-3-ylamino)-ethanol; 2-[(3-Aminopyrazolo[1,5-a]pyrimidin-7-yl)-(2-hydroxy-ethyl)-amino]-ethanol; 2-[(7-Aminopyrazolo[1,5-a]-pyrimidin-3-yl)-(2-hydroxy-ethyl)-amino]-ethanol; 5,6-Dimethylpyrazolo[1,5-a]pyrimidin-3,7-diamin; 2,6-Dimethylpyrazolo[1,5-a]pyrimidin-3,7-diamin; 3-Amino-7-dimethylamino-2,5-dimethylpyrazolo-[1,5-a]pyrimidin sowie ihre physiologisch verträglichen Salze und ihre tautomeren Formen, wenn ein tautomeres Gleichgewicht vorhanden ist.

Ganz besonders bevorzugte Entwicklerkomponenten werden ausgewählt, aus mindestens einer Verbindung aus der Gruppe, die gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diaminopropan-2-ol, Bis-(2-hydroxy-5-aminophenyl)methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxyethyl)phenol und 4-Amino-2-(diethylaminomethyl)phenol, 4,5-Diamino-1-(2-hydroxyethyl)-pyrazol, 2,4,5,6-Tetraaminopyrimidin, 4-Hydroxy-2,5,6-triaminopyrimidin, 2-Hydroxy-4,5,6-triaminopyrimidin, sowie den physiologisch verträglichen Salzen dieser Verbindungen.

Die Entwicklerkomponenten werden bevorzugt in einer Menge von 0,005 bis 20 Gew.-%, vorzugsweise 0,1 bis 5 Gew.-%, jeweils bezogen auf das anwendungsbereite Oxidationsfärbemittel, verwendet.

Kupplerkomponenten bilden im Rahmen der oxidativen Färbung allein keine signifikante Färbung aus, sondern benötigen stets die Gegenwart von Entwicklerkomponenten. Daher ist es erfindungsgemäß bevorzugt, dass bei Verwendung mindestens einer Kupplerkomponente zusätzlich mindestens eine Entwicklerkomponente zum Einsatz kommt.

Kupplerkomponenten im Sinne der Erfindung erlauben mindestens eine Substitution eines chemischen Restes des Kupplers durch die oxidierte Form der Entwicklerkomponente. Dabei bildet sich eine kovalente Bindung zwischen Kuppler- und Entwicklerkomponente aus. Kuppler sind bevorzugt cyclische Verbindungen, die am Cyclus mindestens zwei Gruppen tragen, ausgewählt aus (i) gegebenenfalls substituierten Aminogruppen und/oder (ii) Hydroxylgruppen. Wenn die cyclische Verbindung ein Sechsring (bevorzugt aromatisch) ist, so befinden sich die besagten Gruppen bevorzugt in ortho-Position oder meta-Position zueinander.

Erfindungsgemäße Kupplerkomponenten werden bevorzugt als mindestens eine Verbindung aus einer der folgenden Klassen ausgewählt: m-Aminophenol und/oder dessen Derivate; m-Diaminobenzol und/oder dessen Derivate; o-Diaminobenzol und/oder dessen Derivate, o-Aminophenolderivate; Naphthalinderivate mit mindestens einer Hydroxygruppe; Di- beziehungsweise Trihydroxybenzol und/oder deren Derivate; Pyridinderivate; Pyrimidinderivate, Monohydroxyindol-Derivate und/oder Monoaminoindol-Derivate; Monohydroxyindolin-Derivate und/oder Monoaminoindolin-Derivate; Pyrazolonderivate, wie beispielsweise 1-Phenyl-3-methylpyrazol-5-on; Morpholinderivate, wie beispielsweise 6-Hydroxybenzomorpholin oder 6-Aminobenzomorpholin; Chinoxalinderivate, wie beispielsweise 6-Methyl-1,2,3,4-tetrahydrochinoxalin sowie Gemische aus zwei oder mehrer Verbindungen aus einer oder mehrerer dieser Klassen sind im Rahmen dieser Ausführungsform ebenso erfindungsgemäß.

Bevorzugte m-Aminophenol-Kupplerkomponenten werden ausgewählt aus mindestens einer Verbindung aus der Gruppe, die gebildet wird aus m-Aminophenol, 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)amino-2-methylphenol, 3-Diethylaminophenol, N-Cyclopentyl-3-aminophenol, 1,3-Dihydroxy-5-(methylamino)benzol, 3-Ethylamino-4-methylphenol, 2,4-Dichlor-3-aminophenol und den physiologisch verträglichen Salzen aller vorstehend genannten Verbindungen. Bevorzugte m-Diaminobenzol-Kupplerkomponenten werden ausgewählt aus mindestens einer Verbindung aus der Gruppe, die gebildet wird aus m-Phenylendiamin, 2-(2,4-Diaminophenoxy)ethanol, 1,3-Bis(2,4-diaminophenoxy)propan, 1-Methoxy-2-amino-4-(2'-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}-amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2'-hydroxyethyl)aminobenzol und den physiologisch verträglichen Salzen aller vorstehend genannten Verbindungen. Bevorzugte o-Diaminobenzol-Kupplerkomponenten werden ausgewählt aus mindestens einer Verbindung aus der Gruppe, die gebildet wird aus 3,4-Diaminobenzoesäure und 2,3-Diamino-1-methylbenzol und den physiologisch verträglichen Salzen aller vorstehend genannten Verbindungen. Bevorzugte Dibeziehungsweise Trihydroxybenzole und deren Derivate werden ausgewählt aus mindestens einer Verbindung der Gruppe, die gebildet wird aus Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol. Bevorzugte Pyridinderivate werden ausgewählt aus mindestens einer Verbindung der Gruppe, die gebildet wird aus 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin, 3,5-Diamino-2,6-dimethoxypyridin, 3,4-Diaminopyridin, 2-(2-Methoxyethyl)amino-3-amino-6-methoxypyridin, 2-(4'-Methoxyphenyl)amino-3-aminopyridin, und den physiologisch verträglichen Salzen der vorgenannten Verbindungen. Bevorzugte Naphthalinderivate mit mindestens einer Hydroxygruppe werden ausgewählt aus mindestens einer Verbindung der Gruppe, die gebildet wird aus 1-Naphthol, 2-Methyl-1-naphthol, 2-Hydroxymethyl-1-naphthol, 2-Hydroxyethyl-1-naphthol, 1,3-Dihydroxynaphthalin, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin. Bevorzugte Indolderivate werden ausgewählt aus mindestens einer Verbindung der Gruppe, die gebildet wird aus 4-Hydroxyindol, 6-Hydroxyindol und 7-Hydroxyindol und den physiologisch verträglichen Salzen der vorgenannten Verbindungen. Bevorzugte Indolinderivate werden ausgewählt aus mindestens einer Verbindung der Gruppe, die gebildet wird aus 4-Hydroxyindolin, 6-Hydroxyindolin und 7-Hydroxyindolin und den physiologisch verträglichen Salzen der vorgenannten Verbindungen. Bevorzugte Pyrimidinderivate werden ausgewählt aus mindestens einer Verbindung der Gruppe, die gebildet wird aus 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin und den physiologisch verträglichen Salzen der vorgenannten Verbindungen.

Erfindungsgemäß besonders bevorzugte Kupplerkomponenten werden ausgewählt unter 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)-amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder den physiologisch verträglichen Salzen der vorgenannten Verbindungen.

Die Kupplerkomponenten werden bevorzugt in einer Menge von 0,005 bis 20 Gew.-%, vorzugsweise 0,1 bis 5 Gew.-%, jeweils bezogen auf das anwendungsbereite Oxidationsfärbemittel, verwendet.

Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1 zu 0,5 bis 1 zu 3, insbesondere 1 zu 1 bis 1 zu 2, stehen können.

Als erfindungsgemäße farbverändernde Komponente können in einer weiteren Ausführungsform auch Oxofarbstoffvorprodukte eingesetzt werden. Oxofarbstoffvorprodukte werden bevorzugt als Kombination aus mindestens einer Verbindung, die mindestens eine reaktive Carbonylgruppe enthält (Komponente (Oxo1))
mit mindestens einer Verbindung (Komponente Oxo2), ausgewählt aus (Oxo2a) CH-aciden Verbindungen und/oder aus (Oxo2b) Verbindungen mit primärer oder sekundärer Aminogruppe oder Hydroxygruppe, ausgewählt aus mindestens einer Verbindung der Gruppe, die gebildet wird aus primären oder sekundären aromatischen Aminen, stickstoffhaltigen heterocyclischen Verbindungen und aromatischen Hydroxyverbindungen, eingesetzt.

Bevorzugte reaktive Carbonylverbindungen der Komponente (Oxo1) werden ausgewählt aus der Gruppe, bestehend aus Benzaldehyd und seinen Derivaten, Naphthaldehyd und seinen Derivaten, Zimtaldehyd und seinen Derivaten, 2-Formylmethylen-1,3,3-trimethylindolin (Fischers Aldehyd oder Tribasen Aldehyd), 2-Indolaldehyd, 3-Indolaldehyd, 1-Methylindol-3-aldehyd, 2-Methylindol-3-aldehyd, 2-(1',3',3'-Trimethyl-2-indolinyliden)acetaldehyd, 1-Methylpyrrol-2-aldehyd, Pyridoxal, Antipyrin-4-aldehyd, Furfural, 5-Nitrofurfural, Chromon-3-aldehyd, 3-(5'-Nitro-2'-furyl)acrolein, 3-(2'-Furyl)acrolein und Imidazol-2-aldehyd, 5-(4-Dimethylaminophenyl)penta-2,4-dienal, 5-(4-Diethylaminophenyl)penta-2,4-dienal, 5-(4-Methoxyphenyl)penta-2,4-dienal, 5-(3,4-Dimethoxyphenyl)penta-2,4-dienal, 5-(2,4-Dimethoxyphenyl)penta-2,4-dienal, 5-(4-Piperidinophenyl)penta-2,4-dienal, 5-(4-Morpholinophenyl)penta-2,4-dienal, 5-(4-Pyrrolidinophenyl)penta-2,4-dienal, 5-(4-Dimethylamino-1-naphthyl)penta-3,5-dienal, Piperonal, 6-Nitropiperonal, 2-Nitropiperonal, 5-Nitrovanillin, 2,5-Dinitrosalicylaldehyd, 5-Brom-3-nitrosalicylaldehyd, 3-Nitro-4-formylbenzolsulfonsäure, Salze, deren kationische Komponente 4-Formyl-1-methylpyridinium, 2-Formyl-1-methylpyridinium, 4-Formyl-1-ethylpyridinium, 2-Formyl-1-ethylpyridinium, 4-Formyl-1-benzylpyridinium, 2-Formyl-1-benzylpyridinium, 4-Formyl-1,2-dimethylpyridinium, 4-Formyl-1,3-dimethylpyridinium, 4-Formyl-1-methylchinolinium, 2-Formyl-1-methylchinolinium, 5-Formyl-1-methylchinolinium, 6-Formyl-1-methylchinolinium, 7-Formyl-1-methylchinolinium, 8-Formyl-1-methylchinolinium, 5-Formyl-1-ethylchinolinium, 6-Formyl-1-ethylchinolinium, 7-Formyl-1-ethylchinolinium, 8-Formyl-1-ethylchinolinium, 5-Formyl-1-benzylchinolinium, 6-Formyl-1-benzylchinolinium, 7-Formyl-1-benzylchinolinium, 8-Formyl-1-benzylchinolinium, 5-Formyl-1-allylchinolinium, 6-Formyl-1-allylchinolinium, 7-Formyl-1-allylchinolinium oder 8-Formyl-1-allylchinolinium ist und deren anionisches Gegenion Benzolsulfonat, p-Toluolsulfonat, Methansulfonat, Perchlorat, Sulfat, Chlorid, Bromid, Iodid, Tetrachlorozinkat, Methylsulfat, Trifluormethansulfonat oder Tetrafluoroborat ist, Isatin, 1-Methylisatin, 1-Allylisatin, 1-Hydroxymethylisatin, 5-Chlorisatin, 5-Methoxyisatin, 5-Nitroisatin, 6-Nitroisatin, 5-Sulfoisatin, 5-Carboxyisatin, Chinisatin, 1-Methylchinisatin, sowie beliebigen Gemischen der voranstehenden Verbindungen.

Als CH-acide Verbindungen werden im Allgemeinen solche Verbindungen angesehen, die ein an ein aliphatisches Kohlenstoffatom gebundenes Wasserstoffatom tragen, wobei aufgrund von Elektronen-ziehenden Substituenten eine Aktivierung der entsprechenden Kohlenstoff-WasserstoffBindung bewirkt wird. Prinzipiell sind der Auswahl der CH-aciden Verbindungen keine Grenzen gesetzt, solange nach der Kondensation mit den reaktiven Carbonylverbindungen der Komponente (Oxo1) eine für das menschliche Auge sichtbar farbige Verbindung erhalten wird. Es handelt sich erfindungsgemäß bevorzugt um solche CH-aciden Verbindungen, welche einen aromatischen und/oder einen heterocyclischen Rest enthalten. Der heterocyclische Rest kann wiederum aliphatisch oder aromatisch sein. Besonders bevorzugt werden die CH-aciden Verbindungen ausgewählt aus heterocyclischen Verbindungen, insbesondere kationischen, heterocyclischen Verbindungen.

Die CH-aciden Verbindungen der Oxofarbstoffvorprodukte der Komponente (Oxo2a) werden ganz besonders bevorzugt ausgewählt aus mindestens einer Verbindung der Gruppe, bestehend aus 2-(2-Furoyl)acetonitril, 2-(5-Brom-2-furoyl)acetonitril, 3-(2,5-Dimethyl-3-furyl)-3-oxopropanitril, 2-(2-Thenoyl)acetonitril, 2-(3-Thenoyl)acetonitril, 2-(5-Fluor-2-thenoyl)acetonitril, 2-(5-Chlor-2-thenoyl)acetonitril, 2-(5-Brom-2-thenoyl)acetonitril, 2-(5-Methyl-2-thenoyl)acetonitril, 2-(2,5-Dimethylpyrrol-3-oyl)acetonitril, 2-(1,2,5-Trimethylpyrrol-3-oyl)acetonitril, 1H-Benzimidazol-2-ylacetonitril, 1H-Benzothiazol-2-ylacetonitril, 2-(Pyrid-2-yl)acetonitril, 2,6-Bis-(cyanomethyl)pyridin, 2-(Indol-3-oyl)acetonitril, 2-(2-Methyl-indol-3-oyl)acetonitril, 2-(6-Hydroxy-4,7-dimethoxy-1-benzofuran-5-oyl)acetonitril, 1,2,3,3-Tetramethyl-3H-indolium Iodid, 1,2,3,3-Tetramethyl-3H-indolium p-Toluolsulfonat, 1,2,3,3-Tetramethyl-3H-indolium Methansulfonat, 2,3-Dimethyl-benzothiazolium Iodid, 2,3-Dimethyl-benzothiazolium p-Toluolsulfonat, 1,4-Dimethylchinolinium Iodid, 1,2-Dimethylchinolinium Iodid, 3-Ethyl-2-methyl-benzoxazolium Iodid, 3-Ethyl-2-methyl-benzothiazolium Iodid, 1-Ethyl-4-methyl-chinolinium Iodid, 1-Ethyl-2-methylchinolinium Iodid, 1,2,3-Trimethylchinoxalinium Iodid, 3-Ethyl-2-methyl-benzoxazolium p-Toluolsulfonat, 3-Ethyl-2-methyl-benzothiazolium p-Toluolsulfonat, 1-Ethyl-4-methylchinolinium p-Toluolsulfonat, 1-Ethyl-2-methylchinolinium p-Toluolsulfonat, 1,2,3-Trimethylchinoxalinium p-Toluolsulfonat, 1-Allyl-1,2-dihydro-3,4,6-trimethyl-2-oxopyrimidinium Bromid, 1,2-Dihydro-1-(2-hydroxyethyl)-3,4,6-trimethyl-2-oxopyrimidinium p-Toluolsulfonat, 1,2-Dihydro-1,3,4,6-tetramethyl-2-oxopyrimidinium Chlorid, 1,2-Dihydro-1,3-diethyl-4,6-dimethyl-2-oxopyrimidinium Chlorid, 1,2-Dihydro-1,3-dipropyl-4,6-dimethyl-2-oxopyrimidinium Chlorid, 1-Allyl-1,2-dihydro-3,4,6-trimethyl-2-oxopyrimidinium Hydrogensulfat, 1,2-Dihydro-1-(2-hydroxyethyl)-3,4,6-trimethyl-2-oxopyrimidinium Hydrogensulfat, 1,2-Dihydro-1,3,4,6-tetramethyl-2-oxopyrimidinium Hydrogensulfat, 1,2-Dihydro-1,3-diethyl-4,6-dimethyl-2-oxo-pyrimidinium Hydrogensulfat, 1,2-Dihydro-1,3-dipropyl-4,6-dimethyl-2-oxo-pyrimidinium Hydrogensulfat, 1,2-Dihydro-1,3,4-trimethyl-2-oxo-pyrimidinium Chlorid, 1,2-Dihydro-1,3,4-trimethyl-2-oxo-pyrimidinium Hydrogensulfat, 1,2-Dihydro-1,3-diethyl-4-methyl-2-oxo-pyrimidinium Chlorid, 1,2-Dihydro-1,3-diethyl-4-methyl-2-oxo-pyrimidinium Hydrogensulfat, 1,2-Dihydro-1,3-dipropyl-4-methyl-2-oxo-pyrimidinium Chlorid, 1,2-Dihydro-1,3-dipropyl-4-methyl-2-oxo-pyrimidinium Hydrogensulfat, 1,2-Dihydro-1,3,4,6-tetramethyl-2-thioxo-pyrimidinium Chlorid, 1,2-Dihydro-1,3-diethyl-4,6-dimethyl-2-thioxopyrimidinium Chlorid, 1,2-Dihydro-1,3-dipropyl-4,6-dimethyl-2-thioxo-pyrimidinium Chlorid, 1,2-Dihydro-1,3,4,6-tetramethyl-2-thioxopyrimidinium Hydrogensulfat, 1,2-Dihydro-1,3-dipropyl-4,6-dimethyl-2-thioxopyrimidinium Hydrogensulfat, 1,2-Dihydro-1,3,4-trimethyl-2-thioxopyrimidinium Chlorid, 1,2-Dihydro-1,3,4-trimethyl-2-thioxopyrimidinium Hydrogensulfat, 1,2-Dihydro-1,3-diethyl-4-methyl-2-thioxopyrimidinium Chlorid, 1,2-Dihydro-1,3-diethyl-4-methyl-2-thioxopyrimidinium Hydrogensulfat, 1,2-Dihydro-1,3-dipropyl-4-methyl-2-thioxopyrimidinium Chlorid und 1,2-Dihydro-1,3-dipropyl-4-methyl-2-thioxopyrimidinium Hydrogensulfat.

Des Weiteren kann als Komponente (Oxo2b) mindestens ein Oxidationsfarbstoffvorprodukt mit mindestens einer primären oder sekundären Aminogruppe und/oder mindestens einer Hydroxygruppe verwendet werden. Bevorzugt geeignete Vertreter finden sich unter der Ausführung der Oxidationsfarbstoffvorprodukte. Es ist jedoch erfindungsgemäß bevorzugt, wenn die Verbindungen der Komponente (Oxo2) nur unter CH-aciden Verbindungen ausgewählt werden. Die voranstehend genannten Verbindungen der Komponente (Oxo1) sowie der Komponente (Oxo2) werden, jeweils vorzugsweise in einer Menge von 0,03 bis 65 mmol, insbesondere von 1 bis 40 mmol, bezogen auf 100 g des gesamten Mittels, verwendet.

Weiterhin können die erfindungsgemäßen Mittel als farbverändernde Komponente mindestens einen direktziehenden Farbstoff enthalten. Dabei handelt sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole.

Die direktziehenden Farbstoffe werden jeweils bevorzugt in einer Menge von 0,001 bis 20 Gew.-%, bezogen auf die gesamte Anwendungszubereitung, eingesetzt. Die Gesamtmenge an direktziehenden Farbstoffen beträgt vorzugsweise höchstens 20 Gew.-%. Direktziehende Farbstoffe können in anionische, kationische und nichtionische direktziehende Farbstoffe unterteilt werden.

Als anionische direktziehende Farbstoffe eignen sich insbesondere FD&C Yellow No. 6, Acid Yellow 1, Acid Yellow 3, Acid Yellow 9, Acid Yellow 23, Acid Yellow 36, Acid Yellow 73, Acid Orange 3, Acid Orange 6, Acid Orange 7, Acid Orange 24, Acid Red 4, Acid Red No.14, Acid Red 18, Acid Red 27, Acid Red 33, Acid Red 35, Acid Red 51, Acid Red 52, Acid Red 73, Acid Red 87, Acid Red 92, Acid Red 95, Acid Red 184, Acid Red 195, Pigment Red 57:1, FD&C Red No. 4, Acid Green 25, Acid Green 50, Acid Blue 1, Acid Blue 3, Acid Blue 7, Acid Blue 25, Acid Blue 62, Acid Blue 74, Acid Violet 9, Acid Violet 43, Acid Brown 13, Acid Black 1, Acid Black 52, Food Black No. 1,3',3",5',5"-Tetrabromphenolsulfonphthalein (Bromphenolblau). Bevorzugte anionische direktziehende Farbstoffe sind Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1 und Acid Black 52.

Als kationische direktziehende Farbstoffe eignen sich insbesondere Basic Blue 6, Basic Blue 7, Basic Blue 8, Basic Blue 9, Basic Blue 26, Basic Blue 41, Basic Blue No. 99, Basic Violet 1, Basic Violet 2, Basic Violet 3, Basic Violet 10, Basic Violet 14, Basic Brown 4, Basic Brown 16, 1-[(4-Amino-2-nitrophenyl)azo]-7-trimethylammonio-2-naphthol chlorid, Basic Brown 17, Basic Orange 69, Basic Red 2, Basic Red 22, Basic Red 76, Basic Yellow 2, Basic Yellow 11, Basic Yellow 57, Basic Green 1, Basic Green 4, 1-(2-Morpholiniumpropylamino)-4-hydroxy-9,10-anthrachinon Methylsulfat, 1-[(3-(Dimethylpropylamminium)propyl)amino]-4-methylamino-9,10-anthrachinon Chlorid und direktziehende Farbstoffe, die einen Heterocyclus enthalten, der mindestens ein quaternäres Stickstoffatom aufweist.

Bevorzugte kationische direktziehende Farbstoffe sind dabei kationische Triphenylmethanfarbstoffe, wie beispielsweise Basic Blue 7, Basic Blue 26, Basic Violet 2 und Basic Violet 14, aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie direktziehende Farbstoffe, die einen Heterocyclus enthalten, der mindestens ein quaternäres Stickstoffatom aufweist, wie sie beispielsweise in der EP-A2-998 908 genannt werden. Die Verbindungen, die auch unter den Bezeichnungen Basic Yellow 87, Basic Orange 31 und Basic Red 51 bekannt sind, sind ganz besonders bevorzugte kationische direktziehende Farbstoffe. Die kationischen direktziehenden Farbstoffe, die unter dem Warenzeichen Ariano^{®} vertrieben werden, sind erfindungsgemäß ebenfalls ganz besonders bevorzugte kationische direktziehende Farbstoffe.

Als nichtionische direktziehende Farbstoffe eignen sich insbesondere nichtionische Nitro- und Chinonfarbstoffe und neutrale Azofarbstoffe.

Geeignete blaue Nitrofarbstoffe sind insbesondere 1,4-Bis-[(2-hydroxyethyl)amino]-2-nitrobenzol, HC Blue 2, HC Blue 6, HC Blue 9, HC Blue 10, HC Blue 11, HC Blue 12, HC Blue 13, HC Violet 1, HC Violet 2, 1-(2-Aminoethylamino)-4-[di-(2-hydroxyethyl)amino]-2-nitrobenzol, 4-(Di-(2-hydroxyethyl)amino)-2-nitro-1-phenylaminobenzol. Geeignete rote Nitrofarbstoffe sind insbesondere HC Red 7, 2-Amino-4,6-dinitrophenol und deren Salze, 1,4-Diamino-2-nitrobenzol, HC Red 1, HC Red 13, 1-Amino-4-[(2-hydroxyethyl)-amino]-5-chlor-2-nitrobenzol, HC Red 3, 4-[(2-Hydroxyethyl)-methylamino]-1-(methylamino)-2-nitrobenzol, 1-Amino-4-[(2,3-dihydroxypropyl)amino]-5-methyl-2-nitrobenzol, 1-Amino-4-(methylamino)-2-nitrobenzol, 4-Amino-2-nitro-1-[(prop-2-en-1-yl)amino]-benzol, 4-Amino-3-nitrophenol, 4-[(2-Hydroxyethyl)amino]-3-nitrophenol, HC Orange 1, HC Orange 2, HC Orange 3, HC Red 10, HC Red 11, 2-[(2-Hydroxyethyl)amino]-4,6-dinitrophenol, 4-Ethylamino-3-nitrobenzoesäure, 2-[(4-Amino-2-nitrophenyl)amino]benzoesäure, 2-Chlor-6-ethylamino-4-nitrophenol, 2-Amino-6-chlor-4-nitrophenol, HC Red BN, 2,5-Diamino-6-nitropyridin, 6-Amino-3-[(2-hydroxyethyl)amino]-2-nitropyridin, 3-Amino-6-[(2-hydroxyethyl)amino]-2-nitropyridin, 3-Amino-6-(ethylamino)-2-nitropyridin, 3-[(2-Hydroxyethyl)amino]-6-(methylamino)-2-nitropyridin, 3-Amino-6-(methylamino)-2-nitropyridin, 6-(Ethylamino)-3-[(2-hydroxyethyl)amino]-2-nitropyridin, 1,2,3,4-Tetrahydro-6-nitrochinoxalin, HC Red 14. Geeignete gelbe Nitrofarbstoffe sind insbesondere 1,2-Diamino-4-nitrobenzol, HC Yellow 2, HC Yellow 4, HC Yellow 5, HC Yellow 6, 2-[Di-(2-hydroxyethyl)amino]-5-nitrophenol, 2-[(2-Hydroxyethyl)amino]-1-methoxy-5-nitrobenzol, 2-Amino-3-nitrophenol, 2-Amino-4-nitrophenol, 1-Amino-2-methyl-6-nitrobenzol, 1-(2-Hydroxyethoxy)-3-methylamino-4-nitrobenzol, 2,3-(Dihydroxypropoxy)-3-methylamino-4-nitrobenzol, HC Yellow 9, HC Yellow 10, HC Yellow 11, 1-[(2'-Ureidoethyl)amino]-4-nitrobenzol, 1-Amino-4-[(2-aminoethyl)amino]-5-methyl-2-nitrobenzol, 4-[(2-Hydroxyethyl)amino]-3-nitro-1-methylbenzol, HC Yellow 12, HC Yellow 13, HC Yellow 14, HC Yellow 15, 3-[(2-Hydroxyethyl)amino]-4-methyl-1-nitrobenzol, 4-Chlor-3-[(2-hydroxyethyl)amino]-1-nitrobenzol. Geeignete Chinonfarbstoffe sind insbesondere 1,4-Di-[(2,3-dihydroxypropyl)amino]-9,10-anthrachinon, Disperse Blue 23, Disperse Blue 3, HC Orange 5, Disperse Red 15, 1-Hydroxy-4-[(4-methyl-2-sulfophenyl)amino]-9,10-anthrachinon, Natural Red 4, HC Blue 8, HC Red 8, Disperse Red 11, Disperse Blue 7, Disperse Violet 1, Disperse Violet 4, 2-Hydroxy-3-methoxy-1,4-naphthochinon, 2,5-Dihydroxy-1,4-naphthochinon, 2-Hydroxy-3-methyl-1,4-naphthochinon, HC Red 9, HC Green 1, Natural Brown 7, Natural Orange 6, 1,2-Dihydro-2-(1,3-dihydro-3-oxo-2H-indol-2-yliden)-3H-indol-3-on, 4-{{5-[(2-Hydroxyethyl)amino]-1-methyl-1H-pyrazol-4-yl}imino}-4,5-dihydro-5-[(2-hydroxyethyl)imino]-1-methyl-1H-pyrazol Sulfat (1:1), Hydrat(1:1). Geeignete neutrale Azofarbstoffe sind insbesondere Disperse Red 17, Disperse Black 9, HC Yellow 7, 2,6-Diamino-3-[(pyridin-3-yl)azo]pyridin, Disperse Yellow 3, Disperse Orange 3.

Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)-aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)-amino-5-chlor-2-nitrobenzol, 4-Amino-3-nitrophenol, 1-(2'-Ureidoethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Weiterhin können als direktziehende Farbstoffe auch Naturfarbstoffe eingesetzt werden. Als Naturfarbstoffe im Sinne der Erfindung sind Farbstoffe zu verstehen, die aus Pflanzenteilen gewonnen werden. Insbesondere enthalten die erfindungsgemäßen kosmetischen Mittel mindestens einen Naturfarbstoff, der ausgewählt wird aus der Gruppe, die Diaryloylmethanfarbstoffe, Naphthochinonfarbstoffe, Flavonoidfarbstoffe, Anthrachinonfarbstoffe, Betalainfarbstoffe, Gallotanninfarbstoffe und indigoide Farbstoffe umfasst. Erfindungsgemäß besonders bevorzugte Naturfarbstoffe sind solche, die aus einer Pflanze erhalten wurde, die aus der Gruppe ausgewählt wird, die gebildet wird aus Indigofera tinctoria, Curcuma longa, Lawsonia inermis, Chamomilla recutita, Quercus robur, Rosmarinus officinalis, Rheum undulatum und Beta vulgaris. Besonders bevorzugt ist dabei die Gruppe, die gebildet wird aus Diaryloylmethanfarbstoffen, Naphthochinonfarbstoffen und indigoiden Farbstoffen. Die Naturfarbstoffe werden jeweils bevorzugt in einer Menge von 0,001 bis 20 Gew.-%, bezogen auf die gesamte Anwendungszubereitung, eingesetzt. Die Gesamtmenge an Naturfarbstoffen beträgt vorzugsweise höchstens 20 Gew.-%.

In einer weiteren Ausführungsform der vorliegenden Erfindung wird die farbverändernde Komponente aus Farbstoffvorstufen naturanaloger Farbstoffe ausgewählt.

Als Farbstoffvorstufen naturanaloger Farbstoffe werden bevorzugt solche Indole und Indoline eingesetzt, die mindestens zwei Gruppen ausgewählt aus Hydroxy- und/oder oder Aminogruppen, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. Die Farbstoffvorstufen naturanaloger Farbstoffe werden jeweils bevorzugt in einer Menge von 0,001 bis 5 Gew.-%, bezogen auf die gesamte Anwendungszubereitung, eingesetzt. Die Gesamtmenge an direktziehenden Farbstoffen beträgt vorzugsweise höchstens 3 Gew.-%.

Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin sowie 5,6-Dihydroxyindolin-2-carbonsäure, insbesondere das 5,6-Dihydroxyindolin. Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, insbesondere das 5,6-Dihydroxyindol.

Es ist nicht erforderlich, dass Oxidationsfarbstoffvorprodukte, Oxofarbstoffvorprodukte, direktziehende Farbstoffe oder naturanaloge Farbstoffe jeweils einheitliche Verbindungen darstellen. Vielmehr können, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z. B. toxikologischen, ausgeschlossen werden müssen.

Im Falle der oxidativen Färbungen kann die Entwicklung der Farbe grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Dieser Aufhelleffekt kann unabhängig von der Färbemethode gewünscht sein. Als Oxidationsmittel kommen Persulfate, Peroxodisulfate, Chlorite, Hypochlorite und insbesondere Wasserstoffperoxid oder dessen Anlagerungsprodukte an Harnstoff, Melamin sowie Natriumborat in Frage.

Bevorzugt wird als Oxidationsmittel Wasserstoffperoxid eingesetzt. Bevorzugt beträgt die Menge an Wasserstoffperoxid im anwendungsbereiten Mittel 0,5 bis 12 Gew.-%, bevorzugt 0,8 bis 6 Gew.-%, jeweils bezogen auf das anwendungsbereite Mittel.

Solche Oxidationsmittelzubereitungen sind vorzugsweise wässrige, fließfähige Oxidationsmittelzubereitungen. Dabei sind bevorzugte Zubereitungen dadurch gekennzeichnet, dass die fließfähige Oxidationsmittelzubereitung - bezogen auf ihr Gewicht - 40 bis 90 Gew.-%, vorzugsweise 50 bis 85 Gew.-%, besonders bevorzugt 55 bis 80 Gew.-%, weiter bevorzugt 60 bis 77,5 Gew.-% und insbesondere 65 bis 75 Gew.-% Wasser enthält.

Erfindungsgemäß kann aber das Oxidationsfärbemittel auch zusammen mit einem Katalysator auf das Haar aufgebracht werden, der die Oxidation der Farbstoffvorprodukte, z.B. durch Luftsauerstoff, aktiviert. Solche Katalysatoren sind z. B. bestimmte Enzyme, Iodide, Chinone oder Metallionen. Geeignete Enzyme sind z. B. Peroxidasen, die die Wirkung geringer Mengen an Wasserstoffperoxid deutlich verstärken können. Besonders geeignete Katalysatoren für die Oxidation der Farbstoffvorläufer sind die sogenannten 2-Elektronen-Oxidoreduktasen in Kombination mit den dafür spezifischen Substraten, z. B. Pyranose-Oxidase, Glucose-Oxidase, Glycerin-Oxidase, Pyruvat-Oxidase, Alkohol-Oxidase, Lactat-Oxidase, Tyrosinase-Oxidase, Uricase, Cholinoxidase, Aminosäure-Oxidase. Ein Einsatz bestimmter Metallionen oder-komplexe kann ebenfalls bevorzugt sein, um intensive Färbungen zu erhalten. Geeignete Metallionen sind beispielsweise Zn²⁺, Cu²⁺, Fe²⁺, Fe³⁺, Mn²⁺, Mn⁴⁺, Li⁺, Mg²⁺, Ca²⁺, Ce⁴⁺, V³⁺, Co²⁺, Ru³⁺ und Al³⁺, besonders Zn²⁺, Cu²⁺ und Mn²⁺. Besonders bevorzugte Mittel enthalten diese Metallionen zu 0,0001 bis 2,5 Gew.-%, bezogen auf das Gesamtgewicht des erfindungsgemäßen Mittels.

Das eigentliche Haarfärbemittel wird zweckmäßigerweise unmittelbar vor der Anwendung durch Mischung der Zubereitung des Oxidationsmittels mit der Zubereitung, enthaltend die Farbstoffvorprodukte sowie die erfindungsgemäßen Wirkstoffe, hergestellt. Das entstehende gebrauchsfertige Haarfärbepräparat sollte bevorzugt einen pH-Wert im Bereich von 6 bis 12 aufweisen.

Die erfindungsgemäßen Mittel können zusätzlich auch Blondier- und/oder Bleichmittel enthalten und somit als Mittel bereitgestellt werden, die gleichzeitig färbend und aufhellend wirken. Solche Mittel werden nachfolgend als "Färbemittel", als "aufhellende Färbemittel" oder als "Färbe- und Aufhellmittel" bezeichnet.

Für die starke Aufhellung sehr dunklen Haares ist der alleinige Einsatz von Wasserstoffperoxid oder dessen Anlagerungsprodukten an organische beziehungsweise anorganische Verbindungen oftmals nicht ausreichend. In diesen Fällen wird in der Regel eine Kombination aus Wasserstoffperoxid und Persulfaten bzw. Peroxodisulfaten eingesetzt, woraus eine Steigerung des Aufhellvermögens der Mittel resultiert. Daher kann es, sollte der Verbraucher den Wunsch nach einer sehr starken Blondierung verspüren, in einer weiteren Ausführungsform bevorzugt sein, wenn das erfindungsgemäße Färbemittel zusätzlich mindestens ein anorganisches Persulfatsalz bzw. Peroxodisulfatsalz in dem Mittel zum Aufhellen der keratinischen Fasern enthält. Bevorzugte Peroxodisulfatsalze sind Ammoniumperoxodisulfat, Kaliumperoxodisulfat und Natriumperoxodisulfat. Die Peroxodisulfatsalze können in einer Menge von 0,1 bis 25 Gew.-%, insbesondere in einer Menge von 0,5 bis 15 Gew.-%, bezogen auf das Gesamtgewicht des anwendungsbereiten Mittels, enthalten sein. Der Einsatz von Persulfatsalzen bzw. Peroxodisulfatsalzen erfolgt in der Regel in Form eines gegebenenfalls entstaubten Pulvers oder eines in Form gepressten Formkörpers.

Bei einer Anwendung von zusätzlichen Oxidationsmitteln wird das eigentliche Färbe- und/oder Aufhellmittel zweckmäßigerweise unmittelbar vor der Anwendung durch Mischung einer erfindungsgemäßen Zubereitung, enthaltend in einem kosmetischen Träger mindestens ein Oxidationsfarbstoffvorprodukt, sowie einer Zubereitung, enthaltend das zusätzliche Oxidationsmittel, insbesondere Wasserstoffperoxid, hergestellt. Das erfindungsgemäße Mittel kann dabei das erfindungsgmeäße N-Acyl-Derivat des Chitosans in der Zubereitung mit Oxidationsfarbstoffvorprodukten und/oder in der Oxidationsmittelzubereitung enthalten. Bevorzugt enthält die Zubereitung mit Oxidationsfarbstoffvorprodukten das N-Acyl-Derivat des Chitosans.

Wird eine starke Aufhellung gewünscht, so ist es erfindungsgemäß bevorzugt, wenn zusätzlich eine Blondierzubereitung, enthaltend mindestens ein anorganisches Persulfatsalz bzw. Peroxodisulfatsalz, der Oxidationsmittelzubereitung vor Vermischung mit der erfindungsgemäßen Färbezubereitung beigemischt wird.

Insbesondere bei schwer färbbarem Haar kann die Zubereitung mit den Farbstoffvorprodukten aber auch ohne vorherige Vermischung mit der Oxidationskomponente auf das Haar aufgebracht werden. Nach einer Einwirkdauer von 20 bis 30 Minuten wird dann - gegebenenfalls nach einer Zwischenspülung - die Oxidationskomponente aufgebracht. Nach einer weiteren Einwirkdauer von 10 bis 20 Minuten wird dann gespült und gewünschtenfalls nachshampooniert. Bei dieser Ausführungsform wird gemäß einer ersten Variante, bei der das vorherige Aufbringen der Farbstoffvorprodukte eine bessere Penetration in das Haar bewirken soll, das entsprechende Mittel auf einen pH-Wert von etwa 4 bis 7 eingestellt. Gemäß einer zweiten Variante wird zunächst eine Luftoxidation angestrebt, wobei das aufgebrachte Mittel bevorzugt einen pH-Wert von 7 bis 10 aufweist. Bei der anschließenden beschleunigten Nachoxidation kann die Verwendung von sauer eingestellten Peroxodisulfat-Lösungen als Oxidationsmittel bevorzugt sein.

Weiterhin hat es sich als vorteilhaft erweisen, wenn die Oxidationsmittelzubereitungen mindestens einen Stabilisator oder Komplexbildner enthalten. Besonders bevorzugte Stabilisatoren sind Phenacetin, Alkalibenzoate (Natriumbenzoat) und Salicylsäure. Gebräuchliche und im Rahmen der vorliegenden Erfindung bevorzugte Chelatkomplexbildner sind beispielsweise Polyoxycarbonsäuren, Polyamine, Ethylendiamintetraessigsäure (EDTA), Nitrilotriessigsäure (NTA) und Hydroxyethandiphosphonsäuren bzw. deren Alkalisalze. Erfindungsgemäß bevorzugte Komplexbildner sind stickstoffhaltigen Polycarbonsäuren, insbesondere EDTA, und Phosphonate, vorzugsweise Hydroxyalkan- bzw. Aminoalkanphosphonate und insbesondere 1-Hydroxyethan-1,1-diphosphonat (HEDP) bzw. dessen Di- oder Tetranatriumsalz und/oder Ethylendiamintetramethylenphosphonat (EDTMP) bzw. dessen Hexanatriumsalz und/oder Diethylentriaminpentamethylenphosphonat (DTPMP) bzw. dessen Hepta- oder Octanatriumsalz.

Die Konfektionierung der erfindungsgemäßen Färbemittel unterliegt prinzipiell keinerlei Beschränkungen. Üblicherweise werden die erfindungsgemäßen Mittel als 1-Komponentenmittel konfektioniert, die gegebenenfalls unmittelbar vor der Anwendung mit einer zweiten Zubereitung, enthaltend beispielsweise ein Oxidationsmittel, vermischt werden. Es hat sich aber in einigen Fällen auch als bevorzugt erwiesen, wenn das Produkt als 2-Komponentenmittel konfektioniert ist. Im Rahmen einer bevorzugten Ausführungsform werden die erfindungsgemäßen Mittel daher derart konfektioniert, dass eine der erfindungswesentlichen Komponenten separat verpackt ist. Dabei spielt es erfindungsgemäß zunächst keine Rolle, welche der drei erfindungsgemäßen Komponenten separat verpackt wird; es kann aber bevorzugt sein, die Zubereitung, die einen Extrakt einer Blaualge enthält, bis zur Anwendung separat zu verpacken.

Die Oxidationsmittelzubereitung enthält neben dem eigentlichen Oxidationsmittel weitere Hilfs- und Zusatzstoffe. So hat es sich erfindungsgemäß als bevorzugt erwiesen, wenn die Oxidationsmittelzubereitung mindestens ein Verdickungsmittel enthält. Bezüglich dieser Verdickungsmittel bestehen keine prinzipiellen Einschränkungen. Es können sowohl organische als auch rein anorganische Verdickungsmittel zum Einsatz kommen.

Gemäß einer ersten bevorzugten Ausführungsform handelt es sich bei dem Verdickungsmittel um ein anionisches, synthetisches Polymer, beispielsweise Rheothik^{®}11-80, Aculyn^{®} 33, Aculyn^{®} 22; Structure^{®} 2001, Structure^{®} 3001, Sepigel^{®}305, Simulgel^{®} 600 oder Stabileze^{®} QM.

Gemäß einer zweiten Ausführungsform handelt es sich bei dem Verdickungsmittel um einen kationisches synthetisches Polymer, insbesondere Polyquaternium-37.

In einer dritten bevorzugten Ausführungsform werden natürlich vorkommende Verdickungsmittel eingesetzt. Bevorzugte Verdickungsmittel dieser Ausführungsform sind beispielsweise modifizierte oder unmodifizierte Guargums, Biosaccharidgums mikrobiellen Ursprungs, wie Skleroglucangums oder Xanthangums, Gums aus pflanzlichen Exsudaten, wie Gummi arabicum, Ghatti-Gummi, Karaya-Gummi, Tragant-Gummi, Carrageen-Gummi, Agar-Agar, Johannisbrotkernmehl, Pektine, Alginate, Stärke-Fraktionen und Derivate wie Amylose, Amylopektin und Dextrine, Cellulosederivate, wie beispielsweise Methylcellulose, Carboxyalkylcellulosen und Hydroxyalkylcellulosen, insbesondere Hydroxyethylcellulosen (Cellosize^{®}, Firma Amerchol und Natrosol^{®}, Firma Hercules) und Carboxymethylcellulosen (Blanose^{®}, Firma Aqualon; Aquasorb^{®} und Ambergum^{®}, Firma Hercules; Cellgon^{®}, Firma Montello). Bevorzugt sind weiterhin Stärke und deren Derivate. Erfindungsgemäß einsetzbar sind natürliche, aus Pflanzen gewonnene Stärken und/oder chemisch oder physikalisch modifizierte Stärken.

Aber auch nichtionische, vollsynthetische Polymere, wie beispielsweise Polyvinylalkohol oder Polyvinylpyrrolidinon, sind als erfindungsgemäße Verdickungsmittel einsetzbar. Bevorzugte nichtionische, vollsynthetische Polymere werden beispielsweise von der Firma BASF unter dem Handelsnamen Luviskol^{®} vertrieben. Derartige nichtionische Polymere ermöglichen, neben ihren hervorragenden verdickenden Eigenschaften, auch eine deutliche Verbesserung des sensorischen Gefühls der resultierenden Zubereitungen.

Als anorganische Verdickungsmittel haben sich Schichtsilikate (polymere, kristalline Natriumdisilicate) als besonders geeignet im Sinne der vorliegenden Erfindung erwiesen, wie beispielsweise Bentonit, Montmorillonit oder Hectorit.

Die Konfektionierung der erfindungsgemäßen Färbemittel unterliegt prinzipiell keinerlei Beschränkungen. Üblicherweise werden die erfindungsgemäßen Mittel als 1-Komponentenmittel konfektioniert, die gegebenenfalls unmittelbar vor der Anwendung mit einer zweiten Zubereitung, enthaltend beispielsweise ein Oxidationsmittel, vermischt werden. Es hat sich aber in einigen Fällen auch als bevorzugt erwiesen, wenn das Produkt als 2-Komponentenmittel konfektioniert ist. Im Rahmen einer bevorzugten Ausführungsform werden die erfindungsgemäßen Mittel daher derart konfektioniert, dass eine der erfindungswesentlichen Komponenten separat verpackt ist.

Im Rahmen einer weiteren bevorzugten Ausführungsform handelt es sich bei dem erfindungsgemäßen Mittel um dauerhaft formverändernde Mittel. Diese Mittel bestehen üblicherweise aus zwei beziehungsweise drei Zubereitungen, die nacheinander auf die Fasern aufgebracht werden. Im Weiteren werden folgende Bezeichnungen verwendet:
- "Wellmittel" für die wässrige Zubereitung der keratinreduzierenden Substanz,
- "Zwischenspülung" für die erste Spülung und
- "Fixiermittel" für die wässrige Zubereitung des Oxidationsmittels.

Obwohl das erfindungsgemäße N-Acyl-Derivat des Chitosans prinzipiell in jeder der genannten Zubereitungen eingesetzt werden kann, hat es sich als erfindungsgemäß besonders bevorzugt erwiesen, wenn er im Wellmittel enthalten ist.

Die erfindungsgemäßen Wellmittel enthalten zwingend mindestens ein keratinreduzierende Substanz als formverändernde Komponente, vorzugsweise Mercaptane. Solche Verbindungen sind beispielsweise Thioglykolsäure, Thiomilchsäure, Thioäpfelsäure, Mercaptoethansulfonsäure sowie deren Salze und Ester, Cysteamin, Cystein, Bunte Salze und Salze der schwefligen Säure. Bevorzugt geeignet als formverändernde Komponenten sind die Alkali- oder Ammoniumsalze der Thioglykolsäure und/oder der Thiomilchsäure sowie die freien Säuren. Diese werden in den Wellmitteln bevorzugt in Konzentrationen von 0,5 bis 1,0 mol/kg bei einem pH-Wert von 5 bis 12, insbesondere von 7 bis 9,5, eingesetzt. Zur Einstellung dieses pH-Wertes enthalten die erfindungsgemäßen Wellmittel üblicherweise Alkalisierungsmittel wie Ammoniak, Alkali- und Ammoniumcarbonate und -hydrogencarbonate oder organische Amine wie Monoethanolamin.

Weiterhin können die erfindungsgemäßen Welllotionen wellkraftverstärkende Komponenten enthalten, wie beispielsweise
- heterocyclische Verbindungen wie Imidazol, Pyrrolidin, Piperidin, Dioxolan, Dioxan, Morpholin und Piperazin sowie Derivate dieser Verbindungen. Weiterhin erfindungsgemäß bevorzugte Imidazolderivate sind Biotin, Hydantoin und Benzimidazol. Ganz besonders bevorzugt ist das Imidazol.
- Aminosäuren wie insbesondere Arginin, Citrullin, Histidin, Ornithin und Lysin. Weiterhin haben sich auch Oligopeptide aus durchschnittlich 2-3 Aminosäuren, die einen hohen Anteil (> 50 %, insbesondere > 70 %) an den genannten Aminosäuren haben, als erfindungsgemäß einsetzbar erwiesen. Erfindungsgemäß besonders bevorzugt sind Arginin sowie dessen Salze und argininreiche Oligopeptide.
- Diole wie beispielsweise 2-Ethyl-1,3-hexandiol, 1,3-Butandiol, 1,4-Butandiol, 1,2-Propandiol, 1,3-Propandiol, Neopentylglykol und Ethylenglykol. 1,3-Diole, insbesondere 2-Ethyl-1,3-hexandiol und 1,3-Butandiol.

Bezüglich näherer Informationen zu solchen wellkraftverstärkenden Komponenten wird auf die Druckschriften DE-OS 44 36 065 und EP-B1-363 057 verwiesen, auf deren Inhalt ausdrücklich Bezug genommen wird. Die wellkraftverstärkenden Verbindungen können in den erfindungsgemäßen Welllotionen in Mengen von 0,5 bis 5 Gew.-%, bezogen auf die gesamte Welllotion, enthalten sein. Mengen von 1 bis 4 Gew.-%, im Falle der Diole von 0,5 bis 3 Gew.-%, haben sich als ausreichend erwiesen, weshalb diese Mengen besonders bevorzugt sind.

Zwingender Bestandteil der erfindungsgemäßen Fixiermittel sind Oxidationsmittel, z. B. Natriumbromat, Kaliumbromat, Wasserstoffperoxid, und die zur Stabilisierung wässriger Wasserstoffperoxidzubereitungen üblichen Stabilisatoren. Der pH-Wert solcher wässriger H₂O₂-Zubereitungen, die üblicherweise etwa 0,5 bis 15 Gew.-%, gebrauchsfertig in der Regel etwa 0,5 bis 3 Gew.-%, H₂O₂ enthalten, liegt bevorzugt bei 2 bis 6, insbesondere 2 bis 4. Fixiermittel auf Bromat-Basis enthalten die Bromate üblicherweise in Konzentrationen von 1 bis 10 Gew.-% eingesetzt und der pH-Wert der Lösungen wird auf 4 bis 7 eingestellt. Gleichfalls geeignet sind Fixiermittel auf enzymatischer Basis (z. B. Peroxidasen), die keine oder nur geringe Mengen an Oxidationsmitteln, insbesondere H₂O₂, enthalten.

Die erfindungsgemäßen Wellmittel beziehungsweise Fixiermittel sind üblicherweise einphasig formuliert, eingeschlossen von diesem Begriff sind dabei Systeme, die eine kontinuierliche Phase aufweisen, wie beispielsweise reine Öl-in-Wasser- oder Wasser-in-Öl-Emulsionen.

Es hat sich gezeigt, das erfindungsgemäß auch Zwei- und Mehrphasensysteme bevorzugt sind. Dies sind Systeme, bei denen mindestens zwei separate, kontinuierliche Phasen vorliegen. Beispiele für solche Systeme sind Zubereitungen, die folgende Phasen aufweisen:
- eine wässrige Phase und eine nichtwässrige Phase, die separat voneinander vorliegen
- eine wässrige Phase und zwei nichtwässrige, miteinander nicht mischbare Phasen, die jeweils separat vorliegen
- eine Öl-in-Wasser-Emulsion und eine davon separiert vorliegende nichtwässrige Phase
- eine Wasser-in-Öl-Emulsion und eine davon separiert vorliegende wässrige Phase.

Erfindungsgemäß bevorzugte Mittel zur Farb- und Formveränderung der Haare sind dadurch gekennzeichnet, dass sie einen möglichst neutralen pH-Wert besitzen. Eine weitere bevorzugte Ausführungsform der vorliegenden Erfindung besteht darin, dass das anwendungsbereite Mittel einen pH-Wert zwischen 4,0 und 9,0, bevorzugt zwischen 5,0 und 8,5, insbesondere bevorzugt zwischen 6,0 und 8,0 besitzt. Bei den pH-Werten im Sinne der vorliegenden Erfindung handelt es sich um pH-Werte, die bei einer Temperatur von 22 °C gemessen wurden.

Üblicherweise wird der pH-Wert mit pH-Stellmitteln eingestellt. Zur Einstellung des pH-Werts sind dem Fachmann in der Kosmetik gängige Acidifizierungs- und Alkalisierungsmittel geläufig. Die zur Einstellung des pH-Wertes verwendbaren Alkalisierungsmittel werden typischerweise gewählt aus anorganischen Salzen, insbesondere der Alkali- und Erdalkalimetalle, organischen Alkalisierungsmitteln, insbesondere Aminen, basische Aminosäuren und Alkanolaminen, und Ammoniak. Erfindungsgemäß bevorzugte Acidifizierungsmittel sind Genuss-Säuren, wie beispielsweise Zitronensäure, Essigsäure, Äpfelsäure oder Weinsäure, sowie verdünnte Mineralsäuren. Erfindungsgemäß insbesondere bevorzugte Alkalsierungsmittel sind ausgewählt aus der Gruppe, die gebildet wird aus Natriumhydroxid, Kaliumhydroxid, Calciumhydroxid, Bariumhydroxid, Natriumphosphat, Kaliumphosphat, Natriumsilicat, Kaliumsilicat, Natriumcarbonat, Kaliumcarbonat, 2-Aminoethan-1-ol (Monoethanolamin), 1-Aminopropan-2-ol und 2-Amino-2-methylpropan-1-ol. Ganz besonders bevorzugt sind 2-Aminoethan-1-ol, Natriumhydroxid und Kaliumhydroxid.

Eine bevorzugte Ausführungsform der vorliegenden Erfindung ist weiterhin dadurch gekennzeichnet, dass das Mittel ammoniakarm, bevorzugt ammoniakfrei ist. Die Begriffe "ammoniakarm" bzw. "ammoniakfrei" im Sinne der Erfindung beziehen sich dabei auf die dem Mittel zugesetzte Ammoniakmenge, wobei der Ammoniakzusatz sowohl als wässrige, alkoholische, wässrigalkoholische oder anderweitige Lösung als auch durch Ammoniakgaseinleitung oder durch Zusatz von verflüssigtem Ammoniak erfolgen kann. Der Zusatz von Ammoniak kann aber auch durch Verwendung von Ammonium-Salzen erfolgen, wobei das Ammonium-Kation je nach pH-Wert der Zubereitung im Gleichgewicht mit seiner korrespondierenden Base, dem Ammoniak selbst, steht. Die Begriffe "ammoniakfrei" bzw. "ammoniakarm" im Sinne der Erfindung beziehen sich daher auch auf Mittel, die Ammonium-Salze in den entsprechenden Mengen enthalten. Bevorzugte, ammoniakarme Mittel enthalten weniger als 2 Gew.-%, insbesondere weniger als 0,5 Gew.-% und ganz besonders bevorzugt weniger als 0,1 Gew.-% zugesetzten Ammoniak, jeweils bezogen auf das Gesamtgewicht der Anwendungszubereitung. Ammoniakfrei im Sinne der Erfindung sind solche Mittel, denen kein Ammoniak durch eine der oben beschriebenen Methoden zugesetzt wurde. Solche Mittel sind ganz besonders bevorzugt.

In einer bevorzugten Ausführungsform der vorliegenden Erfindung enthalten die Mittel zusätzlich noch mindestens einen weiteren Wirkstoff, die die pflegenden Eigenschaften der Mittel in synergistischer Weise verbessert. Dieser Wirkstoff wird bevorzugt ausgewählt aus der Gruppe, die gebildet wird aus c1) Silikonderivaten, c2) Polyphenolen und c3) (Pseudo)Ceramiden.

Die Silikonderivate c1) sind, wenn sie in den erfindungsgemäßen Mitteln zugegen sind, vorzugsweise in Mengen von 0,05 bis 5 Gew.-%, vorzugsweise von 0,2 bis 5 Gew.-%, jeweils bezogen auf das anwendungsbereite Mittel, enthalten.

Insbesondere bevorzugt werden die Silikone c1) ausgewählt, aus mindestens einem Vertreter aus der Liste, die gebildet wird aus:
(i) Polyalkylsiloxanen, Polyarylsiloxanen, Polyalkylarylsiloxanen, die flüchtig oder nicht flüchtig, geradkettig, verzweigt oder cyclisch, vernetzt oder nicht vernetzt sind;
(ii) Polysiloxanen, die in ihrer allgemeinen Struktur eine oder mehrere organofunktionelle Gruppen enthalten, wie substituierten oder unsubstituierten aminierten Gruppen; (per)-fluorierten Gruppen; Thiolgruppen; Carboxylatgruppen; hydroxylierten Gruppen; alkoxylierten Gruppen; Acyloxyalkylgruppen; amphoteren Gruppen; Bisulfitgruppen; Hydroxyacylaminogruppen; Carboxygruppen; Sulfonsäuregruppen; und Sulfat- oder Thiosulfatgruppen;
(iii) linearen Polysiloxan(A)-Polyoxyalkylen(B)-Blockcopoylmeren vom Typ (A-B)ₙ mit n > 3;
(iv) gepfropften Silikon polymeren mit nicht silikonhaltigem, organischen Grundgerüst, die aus einer organischen Hauptkette bestehen, welche aus organischen Monomeren gebildet wird, die kein Silikon enthalten, auf die in der Kette sowie gegebenenfalls an mindestens einem Kettenende mindestens ein Polysiloxanmakromer gepfropft wurde;
(v) gepfropften Silikon polymeren mit Polysiloxan-Grundgerüst, auf das nicht silikonhaltige, organische Monomere gepfropft wurden, die eine Polysiloxan-Hauptkette aufweisen, auf die in der Kette sowie gegebenenfalls an mindestens einem ihrer Enden mindestens ein organisches Makromer gepfropft wurde, das kein Silikon enthält, wie beispielsweise das unter der INCI-Bezeichnung Bis-PEG/PPG-20/20 Dimethicone vertriebene Handelsprodukt Abil B 8832 der Firma Degussa;
(vi) oder deren Gemischen.

Erfindungsgemäß geeignet sind kationische Silikonöle wie beispielsweise die im Handel erhältliche Dow Corning 929 Emulsion (enthaltend ein hydroxylamino-modifiziertes Silikon, das als Amodimethicone bezeichnet wird), DC 2-2078 (Hersteller Dow Corning, INCI-Bezeichung: Aminopropyl Phenyl Trimethicone), DC 5-7113 (Dow Corning, INCI-Bezeichnung: Silicone Quaternium 16), SM-2059 (General Electric), SLM-55067 (Wacker) sowie Abil^{®}-Quat 3270 und 3272 (Th. Goldschmidt; diquaternäre Polydimethylsiloxane, Quaternium-80).Weitere geeignete Silikone werden nach der INCI-Deklaration als Trimethylsilylamodimethicone bezeichnet und sind beispielsweise unter der Bezeichnung Q2-7224 (Dow Corning; ein stabilisiertes Trimethylsilylamodimethicon) erhältlich. Weitere geeignete Silikone werden nach der INCI-Deklaration als Amodimethicone, bzw. als funktionalisierte Amodimethicone, wie beispielsweise Bis-(C₁₃-C₁₅-Alkoxy) PG Amodimethicone (DC 8500; Firma Dow Corning), Trideceth-9 PG-Amodimethicone (Silcare Silicone SEA; Firma Clariant) bezeichnet. Auch die nach INCI als Cyclomethicone bezeichneten cyclischen Dimethicone sind erfindungsgemäß mit Vorzug einsetzbar. Mit Vorzug sind die Silikone wasserlöslich. Erfindungsgemäß bevorzugte Mittel der Ausführungsform mit einem Silikon c1) sind dadurch gekennzeichnet, dass das Silikon c1) wasserlöslich ist. Insbesondere bevorzugte wasserlösliche Tenside auf Silikon basis sind ausgewählt aus der Gruppe der Dimethiconcopolyole die bevorzugt alkoxyliert, insbesondere polyethoxyliert oder polypropoxyliert sind. Besonders bevorzugte Dimethiconcopolyole im Sinne der Erfindung sind beispielsweise die kommerziell unter dem Handelsnamen SILWET (Union Carbide Corporation) und Dow Corning (Dow Corning 190 und Dow Corning 193) vertriebenen Produkte.

Polyphenole c2) sind allgemein Verbindungen, die mehr als zwei Phenol- (Polyol-) oder Phenolethergruppen enthalten, die unterschiedlichen Stoffklassen angehören. Die Polyphenole werden bevorzugt ausgewählt aus mindestens einem Vertreter der Gruppe, die gebildet wird, aus c2-1) Hydroxyzimtsäuren, c2-2) 6,7-Dihydroxycumarine, c2-3) Hydroxybenzoesäuren, c2-4) Catechine, c2-5) Leukoanthocyanidine, c2-6) Anthocyanidine, c2-7) Flavanone und c2-8) Flavone und c2-9) Flavonole.

In der Natur treten freie und veretherte Polyphenole beispielsweise in Blütenfarbstoffen (Anthocyanidine, Flavone), in Gerbstoffen (Catechine, Tannine), als Flechten- oder Farn-Inhaltsstoffe (Usninsäure, Acylpolyphenole), in Ligninen und als Gallussäure-Derivate auf. Bevorzugte Polyphenole sind Flavone, Catechine, Usninsäure, und als Tannine die Derivate der Gallussäure, Digallussäure und Digalloylgallussäure. Besonders bevorzugte Polyphenole sind die monomeren Catechine, das heißt die Derivate der Flavan-3-ole, und Leukoanthocyanidine, das heißt die Derivate der Leukoanthocyanidine, die bevorzugt in 5,7,3',4',5'-Stellung phenolische Hydroxygruppen tragen, bevorzugt Epicatechin und Epigallocatechin, sowie die daraus durch Selbstkondensation entstehenden Gerbstoffe. Solche Gerbstoffe werden bevorzugt nicht in isolierter Reinsubstanz, sondern als Extrakte gerbstoffreicher Pflanzenteile eingesetzt, z. B. Extrakte von Catechu, Quebracho und Eichenrinde sowie anderer Baumrinden, Blättern von Grünem Tee (camellia sinensis) und Mate. Ebenfalls besonders bevorzugt sind die Tannine.

Als Ceramide c3) finden bevorzugt die Sphingolipide Verwendung. Als bevorzugte Verbindungen werden erfindungsgemäß die unter den INCI-Bezeichnungen bekannten Verbindungen Ceramide I, Ceramide II, Ceramide 1, Ceramide 2, Ceramide 3, Ceramide 5 und Ceramide 6 als Wirkstoff verwendet. Besonders bevorzugt werden Gemische der Verbindungen mit der Formel (VI) verwendet, die beispielsweise unter dem Handelsnamen SK-Influx^{®} und Ceramide III jeweils von der Firma Degussa Care Specialties erhältlich sind, sowie das Handelsprodukt Ceramide TIC-001, das von der Firma Takasago International Corporation vermarktet wird. Die Verbindungen der Formel (VI) werden bevorzugt in einer Menge von 0,01 bis 1,0 Gew.-% bezogen auf das Gewicht des anwendungsbereiten kosmetischen Mittels verwendet. Außerdem sind Pseudoceramide, wie insbesondere das Pseudoceramid N-(C₈-C₂₂-Acyl)-(C₈-C₂₂-acyl)-hydroxyprolin (Cetyl-hydroxyprolin Palmitamide; Sym Repair 153884, Firma Symrise) erfindungsgemäße Pseudoceramide c3). Vorzugsweise stellen die anwendungsbereiten Färbemittel fließfähige Zubereitungen dar.

Bevorzugt enthalten die fließfähigen Zubereitungen zusätzlich als oberflächenaktive Substanz ein Emulgator bzw. ein Tensid, wobei oberflächenaktive Substanzen je nach Anwendungsgebiet als Tenside oder als Emulgatoren bezeichnet werden und aus anionischen, kationischen, amphoteren, zwitterionischen und nichtionischen Tensiden und Emulgatoren ausgewählt sind. In einer bevorzugten Ausführungsform können Mittel, enthaltend nicht-ionische, zwitterionische und/oder kationische Tenside sowie deren Mischungen, bevorzugt sein.

Ferner können die erfindungsgemäßen Mittel weitere Wirk-, Hilfs- und Zusatzstoffe enthalten, wie beispielsweise nichtionische Polymere; kationische Polymere; zwitterionische und amphotere Polymere; anionische Polymere; Strukturanten wie Glucose, Maleinsäure und Milchsäure; haarkonditionierende Verbindungen wie Phospholipide, beispielsweise Sojalecithin, Ei-Lecitin; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; Entschäumer wie Silikone, bevorzugt Dimethicon; Farbstoffe/Pigmente zum Anfärben des Mittels; Antischuppenwirkstoffe wie Piroctone Olamine, Zink Omadine und Climbazol; Lichtschutzmittel, insbesondere derivatisierte Benzophenone, Zimtsäure-Derivate und Triazine; Wirkstoffe wie Panthenol, Pantothensäure, Pantolacton, Allantoin, Pyrrolidinoncarbonsäuren und deren Salze sowie Bisabolol; Vitamine, Provitamine und Vitaminvorstufen, insbesondere solche der Gruppen A, B₃, B₅, B₆, C, E, F und H; Pflanzenextrakte; Konsistenzgeber wie Zuckerester, Polyolester oder Polyolalkylether; Fette und Wachse wie Bienenwachs, Montanwachs und Paraffine; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP- und Styrol/Acrylamid-Copolymere; Perlglanzmittel wie Ethylenglykolmono- und -distearat sowie PEG-3-distearat; Treibmittel wie Propan-Butan-Gemische, N₂O, Dimethylether, CO₂ und Luft und Antioxidantien.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Erfindungsgemäß bevorzugte Mittel zur Farbveränderung sind dadurch gekennzeichnet, dass das Mittel unmittelbar vor der Anwendung durch Vermischen mindestens zweier Zubereitungen hergestellt wird, wobei die mindestens zwei Zubereitungen in mindestens zwei getrennt konfektionierten Containern bereitgestellt werden und wobei ein Container ein Färbemittel, welches in einem kosmetischen Träger mindestens ein Oxidationsfarbstoffvorprodukt sowie mindestens ein N-Acyl-Derivat des Chitosans, das aus Glucosamin, N-Acetylglucosamin und N-Succinylglucosamin aufgebaut ist, und mindestens einen Wirkstoff, der ausgewählt wird aus der Gruppe, die gebildet wird aus L-Serin, D-Serin und D/L-Serin, enthält und einen pH-Wert zwischen 5,0 und 8,5, bevorzugt zwischen 6,0 und 8,0 besitzt, enthält, und ein weiterer Container eine Oxidationsmittelzubereitung, enthaltend mindestens ein Oxidationsmittel, enthält.

Ein zweiter Gegenstand der vorliegenden Erfindung ist ein Verfahren zur Farb- und/oder dauerhaften Formveränderung keratinischer Fasern, bei dem ein erfindungsgemäßes Mittel gemäß obiger Vorgaben auf das Haar aufgetragen wird, für eine Einwirkzeit von 2 bis 45 Minuten, bevorzugt von 15 bis 30 Minuten auf dem Haar belassen wird, und anschließend das Haar ausgespült wird.

Eine weitere bevorzugte Ausführungsform des erfindungsgemäßen Verfahrens zur Farbveränderung keratinischer Fasern liegt vor, wenn eine Zusammensetzung in einem kosmetischen Träger, enthaltend mindestens ein Oxidationsfarbstoffvorprodukt sowie mindestens ein N-Acyl-Derivat des Chitosans, das aus Glucosamin, N-Acetylglucosamin und N-Succinylglucosamin aufgebaut ist, und mindestens einen Wirkstoff, der ausgewählt wird aus der Gruppe, die gebildet wird aus L-Serin, D-Serin und D/L-Serin, mit einer Oxidationsmittelzubereitung, enthaltend Wasserstoffperoxid, zu einer homogenen Zusammensetzung vermischt, diese auf das Haar aufgebracht wird, für eine Einwirkzeit von 2 bis 45 Minuten, bevorzugt von 15 bis 30 Minuten auf dem Haar belassen wird, und anschließend das Haar ausgespült wird.

Eine weitere bevorzugte Ausführungsform des erfindungsgemäßen Verfahrens zur dauerhaften Formveränderung keratinischer Fasern liegt vor, wenn eine Zusammensetzung in einem kosmetischen Träger, enthaltend mindestens eine keratinreduzierende Substanz sowie mindestens ein N-Acyl-Derivat des Chitosans, das aus Glucosamin, N-Acetylglucosamin und N-Succinylglucosamin aufgebaut ist, und mindestens einen Wirkstoff, der ausgewählt wird aus der Gruppe, die gebildet wird aus L-Serin, D-Serin und D/L-Serin, auf das Haar aufgebracht wird, für eine Einwirkzeit von 2 bis 45 Minuten, bevorzugt von 15 bis 30 Minuten auf dem Haar belassen wird, und anschließend gegebenenfalls das Haar ausgespült wird. Anschließend wird ein Fixiermittel, enthaltend mindestens ein Oxidationsmittel, vorzugsweise Wasserstoffperoxid, sowie vorzugsweise zusätzlich mindestens einen Stabilisator oder Komplexbildner, auf das Haar aufgebracht, für eine Einwirkzeit von 2 bis 45 Minuten, bevorzugt von 15 bis 30 Minuten auf dem Haar belassen wird, und anschließend das Haar ausgespült wird.

Die Anwendungstemperaturen können in einem Bereich zwischen 15 und 45 °C liegen. Bezüglich weiterer bevorzugter Ausführungsformen der erfindungsgemäßen Verfahren gilt mutatis mutandis das zu den erfindungsgemäßen Mitteln Gesagte.

Wie bereits erwähnt, können die erfindungsgemäßen Mittel auch direkt vor der Anwendung aus zwei oder mehreren getrennt verpackten Zubereitungen hergestellt werden. Dies bietet sich insbesondere zur Trennung inkompatibler Inhaltsstoffe an, um eine vorzeitige Reaktion zu vermeiden. Eine Auftrennung in Mehrkomponentensysteme bietet sich insbesondere dort an, wo Inkompatibilitäten der Inhaltsstoffe zu erwarten oder zu befürchten sind. Das anwendungsbereite Mittel wird bei solchen Systemen vom Verbraucher direkt vor der Anwendung durch Vermischen der Komponenten hergestellt. Ein Färbe- und/oder Aufhellungsmittel, bei dem die Oxidationsfarbstoffvorprodukte zunächst getrennt von der Oxidationsmittelzubereitung, enthaltend bevorzugt Wasserstoffperoxid, vorliegen, ist dabei bevorzugt.

Ein vierter Gegenstand der vorliegenden Erfindung ist daher eine Mehrkomponentenverpackungseinheit (Kit-of-Parts), enthaltend mindestens zwei getrennt konfektionierte Container, wobei ein Container mindestens ein Container eine farb- und/oder formverändernde Verbindung, mindestens ein N-Acyl-Derivat des Chitosans, das aus Glucosamin, N-Acetylglucosamin und N-Succinylglucosamin aufgebaut ist, und mindestens einen Wirkstoff, der ausgewählt wird aus der Gruppe, die gebildet wird aus L-Serin, D-Serin und D/L-Serin, enthält und ein Container eine Oxidationsmittelzusammensetzung, enthaltend mindestens ein chemisches Oxidationsmittel, insbesondere Wasserstoffperoxid, enthält

Eine bevorzugte Ausführungsform dieses Gegenstand der vorliegenden Erfindung ist daher eine Mehrkomponentenverpackungseinheit (Kit-of-Parts), enthaltend mindestens zwei getrennt konfektionierte Container, wobei ein Container eine Färbemischung in einem kosmetischen Träger, enthaltend mindestens eine farbgebende Komponente, insbesondere mindestens ein Oxidationsfarbstoffvorprodukt, mindestens ein N-Acyl-Derivat des Chitosans, das aus Glucosamin, N-Acetylglucosamin und N-Succinylglucosamin aufgebaut ist, und mindestens einen Wirkstoff, der ausgewählt wird aus L-Serin, D-Serin und D/L-Serin, und ein Container eine Oxidationsmittelzusammensetzung, enthaltend mindestens ein chemisches Oxidationsmittel, insbesondere Wasserstoffperoxid, enthält. Wird eine besonders starke Aufhellwirkung durch Einsatz von Persulfatsalzen bzw. Peroxodisulfatsalzen gewünscht, ist es erfindungsgemäß bevorzugt, diese der erfindungsgemäßen Mehrkomponentenverpackungseinheit (Kit-of-Parts) in Form eines gegebenenfalls entstaubten Pulvers oder eines in Form gepressten Formkörpers als separat verpackte, zusätzliche Komponente beizufügen.

Eine weitere bevorzugte Ausführungsform des Erfindungsgegenstands ist eine Mehrkomponentenverpackungseinheit (Kit-of-Parts), enthaltend mindestens zwei getrennt konfektionierte Container, wobei ein Container eine Formveränderungsmischung in einem kosmetischen Träger, enthaltend mindestens eine formverändernde Komponente, mindestens ein N-Acyl-Derivat des Chitosans, das aus Glucosamin, N-Acetylglucosamin und N-Succinylglucosamin aufgebaut ist und mindestens einen Wirkstoff, der ausgewählt wird aus L-Serin, D-Serin und D/L-Serin, und ein Container eine Oxidationsmittelzusammensetzung, enthaltend mindestens ein chemisches Oxidationsmittel, insbesondere Wasserstoffperoxid, enthält.

Bevorzugt enthält die Mehrkomponentenverpackungseinheit (Kit-of-Parts) zusätzlich eine Gebrauchsanleitung. Darüber hinaus kann es bevorzugt sein, wenn weiterhin eine Applikationshilfe, wie beispielsweise ein Kamm oder ein Pinsel, und/oder eine persönliche Schutzausrüstung, wie Einweg-Handschuhe dem Kit beigefügt ist. Bezüglich weiterer bevorzugter Ausführungsformen der Mehrkomponentenverpackungseinheit (Kit-of-Parts) gilt mutatis mutandis das zu den erfindungsgemäßen Mitteln Gesagte.

Ein weiterer Gegenstand der Erfindung ist die Verwendung der Mittel des ersten Erfindungsgegenstandes zur Verbesserung des Glanzes der Haare bei der Farb- und/oder dauerhaften Formveränderung menschlicher Haare. Ein weiterer Gegenstand der Erfindung ist die Verwendung der Mittel des ersten Erfindungsgegenstandes zur Verbesserung des Feuchtigkeitsgehaltes der Haare bei der Farb- und/oder dauerhaften Formveränderung menschlicher Haare.

Bezüglich weiterer bevorzugter Ausführungsformen der erfindungsgemäßen Verwendungen gilt mutatis mutandis das zu den erfindungsgemäßen Mitteln Gesagte.

### Beispiele

Eine erfindungsgemäße Färbecreme wurde aus folgenden Inhaltsstoffen hergestellt

| | **E1 [Gew.-%]** |
|---|---|
| Hydrasens¹ | 0,1 |
| Carbomer | 1,3 |
| Kaliumhydroxid (50%) | 2,0 |
| Emulgade F² | 3,0 |
| Disodium Cocoamphodiacetate | 5,0 |
| Farbpulvermischung* | 2,3 |
| Ascorbinsäure | 0,1 |
| Natriumsulfit, vuasserfrei, 96% | 0,2 |
| Phenoxyethanol | 0,9 |
| L-Serin | 1,0 |
| Parfum | 0,2 |
| Wasser | ad 100 |

| | |
|---|---|
| *Farbpulvermischung aus verschiedenen Oxidationsfarbstoffvorprodukten ¹Hydrasens^{®} N-Succinylsuccinamid (INCI-Bezeichnung: Water, Chitosan succinamide, Sodium lactate, Disodium succinate, Propylen glycol, Phenoxyethanol, Methylparaben) (Soliance) ²Emulgade^{®} F C₁₆-C₁₈-Fettalkohol; ethoxyliertes Rizinusöl (40 EO); C₁₆-C₁₈-Fettalkoholsulfat, Natriumsalz (INCI-Bezeichnung: Cetearyl alcohol, PEG-40 castor oil, Sodium Cetearyl Sulfate) (Cognis) | |

Die Färbecreme wurde im Gewichtsverhältnis 1:1 mit einer wie folgt zusammengesetzten Entwicklerdispersion ausgemischt. Der Mischungs-pH-Wert betrug 7.2.

| **Rohstoff** | **Gew.-%** |
|---|---|
| Natrium benzoat | 0,1 |
| Dipicolinsäure | 0,1 |
| Dinatriumpyrophosphat | 0,1 |
| Kaliumhydroxid (50%) | 0,3 |
| 1,2-Propandiol | 1,5 |
| HEDP, 60% | 0,4 |
| Paraffinum Liquidum | 0,3 |
| Genamin STAC¹ | 0,4 |
| Eumulgin B 2² | 1,0 |
| Cetearyl Alcohol | 3,4 |
| Parfum | q.s. |
| Wasserstoffperoxid (50 %) | 8 |
| Wasser | ad 100 |

| | |
|---|---|
| ¹Genamin^{®} STAC Trimethylstearylammoniumchlorid (INCI-Bezeichnung: Steartrimonium chloride) (Clariant) ²Eumulgin^{®} B 2 C₁₆-C₁₈-Fettalkohol, ethoxyliert (20 EO) (INCI-Bezeichnung: Ceteareth-20) (Cognis) | |

Für den Färbung wurde auf Strähnen menschlichen (Codes: Kerling ENH 10/0 bzw. Kerling ENH 6/0) von ca. 0,7 g Gewicht jeweils die 4-fache Menge der fertigen Anwendungsmischung appliziert. Nachdem die Strähnen für 20 Minuten bei 32 °C gefärbt wurden, wurden sie mit einem handelsüblichen Shampoo gewaschen und mit einem Föhn getrocknet.

## Patentansprüche

1. Mittel zur Farb- und/oder dauerhaften Formveränderung keratinischer Fasern, insbesondere menschlicher Haare, enthaltend in einem kosmetischen Träger mindestens eine farb- und / oder formverändernde Verbindung, **dadurch gekennzeichnet, dass** es zusätzlich mindestens ein N-Acyl-Derivat des Chitosans und mindestens einen Wirkstoff, der ausgewählt wird aus der Gruppe, die gebildet wird aus L-Serin, D-Serin und D/L-Serin, enthält, wobei das N-Acyl-Derivat des Chitosans aus Glucosamin, N-Acetylglucosamin und N-Succinylglucosamin aufgebaut ist.

2. Mittel nach Anspruch 1, **dadurch gekennzeichnet, dass** das N-Acyl-Derivat des Chitosans ein Molekulargewicht von 100.000 bis 500.000 g/mol besitzt.

3. Mittel nach einem der Ansprüche 1 bis 2, **dadurch gekennzeichnet, dass** das Mittel zusätzlich mindestens einen Extrakt aus einer Pflanze der Gattung Aloe enthält.

4. Mittel nach einem der Ansprüche 1 bis 3, **dadurch gekennzeichnet, dass** die farbgebende Komponente ausgewählt wird, (1) aus mindestens einem Oxidationsfarbstoffvorprodukt und/oder (2) aus Oxofarbstoffvorprodukten und/oder (3) aus mindestens einem direktziehenden Farbstoff und/oder (4) aus mindestens einer Vorstufe naturanaloger Farbstoffe und/oder (5) mindestens einem Naturfarbstoff.

5. Mittel nach einem der Ansprüche 1 bis 4, **dadurch gekennzeichnet, dass** das anwendungsbereite Mittel einen pH-Wert zwischen 4,0 und 9,0, bevorzugt zwischen 5,0 und 8,5, insbesondere bevorzugt zwischen 6,0 und 8,0 besitzt.

6. Mittel nach einem Ansprüche 1 bis 5, **dadurch gekennzeichnet, dass** es zusätzlich ein Alkalisierungsmittel enthält, das ausgewählt ist aus der Gruppe, die gebildet wird aus Natriumhydroxid, Kaliumhydroxid, Calciumhydroxid, Bariumhydroxid, Natriumphosphat, Kaliumphosphat, Natriumsilicat, Kaliumsilicat, Natriumcarbonat, Kaliumcarbonat, 2-Aminoethan-1-ol (Monoethanolamin), 1-Aminopropan-2-ol und 2-Amino-2-methylpropan-1-ol.

7. Mittel nach einem der Ansprüche 1 bis 6, **dadurch gekennzeichnet, dass** es anionische Tenside in einem Gesamtgehalt von weniger als 1,0 Gew.-%, bevorzugt weniger als 0,5 Gew.-%, jeweils bezogen auf das Gesamtgewicht der Anwendungszubereitung, enthält.

8. Mittel nach einem der Ansprüche 1 bis 7, **dadurch gekennzeichnet, dass** unmittelbar vor der Anwendung durch Vermischen mindestens zweier Zubereitungen hergestellt wird, wobei die mindestens zwei Zubereitungen in mindestens zwei getrennt konfektionierten Containern bereitgestellt werden und wobei ein Container ein Färbemittel (a), welches in einem kosmetischen Träger mindestens ein Oxidationsfarbstoffvorprodukt sowie mindestens ein N-Acyl-Derivat des Chitosans, das aus Glucosamin, N-Acetylglucosamin und N-Succinylglucosamin aufgebaut ist, und mindestens einen Wirkstoff, der ausgewählt wird aus der Gruppe, die gebildet wird aus L-Serin, D-Serin und D/L-Serin, enthält und einen pH-Wert von 5,0 bis 8,5, bevorzugt 6,0 bis 8,0 besitzt, enthält, und ein weiterer Container eine Oxidationsmittelzubereitung (b), enthaltend mindestens ein Oxidationsmittel, enthält.

9. Verfahren zur Behandlung menschlicher Haare, bei dem ein Mittel nach einem der Ansprüche 1 bis 8 auf das Haar aufgetragen wird, für eine Einwirkzeit von 2 bis 45 Minuten, bevorzugt von 15 bis 30 Minuten auf dem Haar belassen wird, und anschließend das Haar ausgespült wird.

10. Verwendung eines Mittels gemäß einem der Ansprüche 1 bis 8 zur Verbesserung des Glanzes der Haare bei der Farb- und/oder dauerhaften Formveränderung menschlicher Haare.

11. Verwendung eines Mittels gemäß einem der Ansprüche 1 bis 8 zur Verbesserung des Feuchtigkeitsgehaltes der Haare bei der Farb- und/oder dauerhaften Formveränderung menschlicher Haare.

12. Kit-of-Parts, enthaltend mindestens zwei getrennt konfektionierte Container, wobei ein Container mindestens ein Container eine farb- und/oder formverändernde Verbindung, mindestens ein N-Acyl-Derivat des Chitosans, das aus Glucosamin, N-Acetylglucosamin und N-Succinylglucosamin aufgebaut ist, und mindestens einen Wirkstoff, der ausgewählt wird aus der Gruppe, die gebildet wird aus L-Serin, D-Serin und D/L-Serin, enthält und ein Container eine Oxidationsmittelzusammensetzung, enthaltend mindestens ein chemisches Oxidationsmittel, insbesondere Wasserstoffperoxid, enthält.

## Claims

1. An agent for changing the color and/or permanently changing the shape of keratin fibers, in particular human hair, said agent containing in a cosmetic carrier at least one color-changing and/or shape-changing compound, **characterized in that** said agent additionally contains at least one N-acyl derivative of chitosan and at least one active ingredient selected from the group formed by L-serine, D-serine and D/L-serine, the N-acyl derivative of the chitosan being composed of glucosamine, N-acetylglucosamine and N-succinylglucosamine.

2. The agent according to claim 1, **characterized in that** the N-acyl derivative of chitosan has a molecular weight of from 100,000 to 500,000 g/mol.

3. The agent according to one of claims 1 to 2, **characterized in that** the agent additionally contains at least one extract of a plant of the genus aloe.

4. The agent according to one of claims 1 to 3, **characterized in that** the color-imparting component is selected (1) from at least one oxidation dye precursor and/or (2) from oxo dye precursors and/or (3) from at least one substantive dye and/or (4) from at least one precursor of nature-analogous dyes and/or (5) at least one natural dye

5. The agent according to one of claims 1 to 4, **characterized in that** the ready-to-apply agent has a pH of between 4.0 and 9.0, preferably between 5.0 and 8.5, in particular between 6.0 and 8.0.

6. The agent according to one of claims 1 to 5, **characterized in that** it additionally contains an alkalizing agent selected from the group formed by sodium hydroxide, potassium hydroxide, calcium hydroxide, barium hydroxide, sodium phosphate, potassium phosphate, sodium silicate, potassium silicate, sodium carbonate, potassium carbonate, 2-aminoethan-1-ol (monoethanolamine), 1-aminopropan-2-ol and 2-amino-2-methylpropan-1-ol.

7. The agent according to one of claims 1 to 6, **characterized in that** it contains anionic surfactants in a total content of less than 1.0 wt.%, preferably less than 0.5 wt.%, in each case based on the total weight of the preparation for use.

8. The agent according to one of claims 1 to 7, **characterized in that** it is produced immediately before application by mixing at least two preparations, the at least two preparations being provided in at least two separately packaged containers, and one container containing a coloring agent (a) which contains, in a cosmetic carrier, at least one oxidation dye precursor and at least one N-acyl derivative of chitosan that is composed of glucosamine, N-acetylglucosamine and N-succinylglucosamine, and at least one active ingredient selected from the group formed by L-serine, D-serine and D/L-serine and has a pH of from 5.0 to 8.5, preferably from 6.0 to 8.0, and a further container containing an oxidizing agent preparation (b) containing at least one oxidizing agent.

9. A method for treating human hair, in which method an agent according to one of claims 1 to 8 is applied to the hair, is left on the hair for a contact time of from 2 to 45 minutes, preferably from 15 to 30 minutes, and is subsequently rinsed out of the hair.

10. The use of an agent according to one of claims 1 to 8 for improving the shine of the hair when changing the color and/or permanently changing the shape of human hair.

11. The use of an agent according to one of claims 1 to 8 for improving the moisture content of the hair when changing the color and/or permanently changing the shape of human hair.

12. A kit of parts containing at least two separately packaged containers, wherein one container at least one container contains a color-changing and/or shape-changing compound, at least one N-acyl derivative of chitosan that is composed of glucosamine, N-acetylglucosamine and N-succinylglucosamine, and at least one active ingredient selected from the group formed by L-serine, D-serine and D/L serine, and one container contains an oxidizing agent preparation containing at least one chemical oxidizing agent, in particular hydrogen peroxide.

## Revendications

1. Agent de coloration et/ou de mise en forme permanente de fibres de kératine, en particulier de cheveux humains, comprenant dans un support cosmétique au moins un composé de coloration et/ou de mise en forme, **caractérisé en ce qu'**il contient en outre au moins un dérivé N-acylé du chitosane et au moins un principe actif choisi dans le groupe formé par la L-sérine, la D-sérine et la D/L-sérine, le dérivé N-acylé du chitosane est obtenu à partir de la glucosamine, de la N-acétylglucosamine et de la N-succinylglucosamine.

2. Agent selon la revendication 1, **caractérisé en ce que** le dérivé N-acylé du chitosane a un poids moléculaire de 100000 à 500000 g/mol.

3. Agent selon l'une des revendications 1 à 2, **caractérisé en ce que** l'agent contient en outre au moins un extrait d'une plante du genre Aloès.

4. Agent selon l'une des revendications 1 à 3, **caractérisé en ce que** le composé colorant est choisi (1) parmi au moins un précurseur de colorant d'oxydation et/ou (2) à partir de des précurseurs de colorant oxo et/ou (3) à partir d'au moins un colorant direct et/ou (4) à partir d'au moins un précurseur de colorant quasi-naturel et/ou (5) d'au moins un colorant naturel.

5. Agent selon l'une des revendications 1 à 4, **caractérisé en ce que** l'agent prêt à l'emploi a une valeur de pH comprise entre 4,0 et 9,0, de préférence entre 5,0 et 8,5, de manière en particulièrement préférée entre 6,0 et 8,0.

6. Agent selon l'une des revendications 1 à 5, **caractérisé en ce qu'**il contient en outre un agent alcalinisant choisi dans le groupe formé par l'hydroxyde de sodium, l'hydroxyde de potassium, l'hydroxyde de calcium, l'hydroxyde de baryum, le phosphate de sodium, le phosphate de potassium, le silicate de sodium, le silicate de potassium , le carbonate de sodium, le carbonate de potassium, le 2-aminoéthan-1-ol (monoéthanolamine), le 1-aminopropan-2-ol et le 2-amino-2-méthylpropan-1-ol.

7. Agent selon l'une des revendications 1 à 6, **caractérisé en ce qu'**il contient des tensioactifs anioniques à une teneur totale inférieure à 1,0% en poids, de préférence inférieure à 0,5% en poids, à chaque fois par rapport au poids total de la préparation prête à l'emploi.

8. Agent selon l'une des revendications 1 à 7, **caractérisé en ce qu'**il est produit immédiatement avant l'utilisation par mélange d'au moins deux compositions, les au moins deux compositions étant présentées dans au moins deux récipients séparés et un récipient contenant un colorant (a), qui contient dans un support cosmétique au moins un précurseur de colorant d'oxydation et au moins un dérivé N-acylé du chitosane obtenu à partir de la glucosamine, de la N-acétylglucosamine et de la N-succinylglucosamine, et au moins un principe actif choisi dans le groupe formé par la L-sérine, la D-sérine et la D/L-sérine, et ayant un pH de 5,0 à 8,5, de préférence de 6,0 à 8,0, et un autre récipient contenant une composition d'agent d'oxydation (b) incluant au moins un agent oxydant.

9. Procédé de traitement de cheveux humains, dans lequel un agent selon l'une des revendications 1 à 8 est appliqué sur les cheveux pendant un temps d'action de 2 à 45 minutes, de préférence de 15 à 30 minutes puis les cheveux sont rincés.

10. Utilisation d'un agent selon l'une des revendications 1 à 8, pour améliorer l'éclat des cheveux lors de la coloration et/ou de la mise en forme permanente de cheveux humains.

11. Utilisation d'un agent selon l'une des revendications 1 à 8 pour améliorer la teneur en humidité des cheveux lors de la coloration et/ou de la mise en forme permanente de cheveux humains.

12. Kit-of-parts contenant au moins deux récipients séparés, un récipient contenant au moins un composé de coloration et/ou de mise en forme, au moins un dérivé N-acyle du chitosane obtenu à partir de la glucosamine, de la N-acétylglucosamine et de la N-succinylglucosamine, et au moins un principe actif choisi dans le groupe formé par la L-sérine, la D-sérine et la D/L-sérine, et un récipient contenant une composition d'agent d'oxydation incluant au moins un agent d'oxydation chimique, en particulier du peroxyde d'hydrogène.
